# EUROPEAN PATENT APPLICATION

(11) **EP 4 721 745 A1**
(43) Date of publication of application: **08.04.2026**
(21) Application number: 24814565.8
(22) Date of filing: 31.05.2024
(51) Int. Cl.: A61K 31/575, A61K 31/4745, A61P 1/16

(54) **COMBINED PRODUCT, SALT AND USE THEREOF**

(30) Priority: 31.05.2023 CN 202310632253; 17.04.2024 CN 202410474354
(71) Applicant: Shanghai Henlius Biotech, Inc., Shanghai 201210 (CN); Shanghai Henlius Biologics Co., Ltd., Shanghai 201616 (CN)
(72) Inventor: SHAN, Yongqiang, Shanghai 201210 (CN); LV, Zhiliang, Shanghai 201210 (CN); FENG, Teng, Shanghai 201210 (CN)
(74) Representative: Rentsch Partner AG
(86) International application number: PCT/CN2024/096519
(87) International publication number: WO 2024/245370

(57) **Abstract**

A combined product, a pharmaceutical composition, a salt form and the use thereof in the treatment and/or prevention of diseases.

## Description

### TECHNICAL FIELD

The present disclosure belongs to the field of pharmaceuticals, and relates to a novel combination product, a pharmaceutical composition, a salt form, and use thereof in the treatment and/or prevention of a disease.

### BACKGROUND

Liver diseases are generally classified as acute or chronic based on the duration of the disease. Liver diseases may be caused by infections, injuries, exposure to drugs or toxic compounds, alcohol, impurities in food, abnormal accumulation of normal substances in the blood, autoimmune processes, genetic defects (e.g., hemochromatosis), or unknown reasons. Liver diseases are the leading cause of death worldwide.

Primary biliary cirrhosis (PBC) and primary sclerosing cholangitis (PSC) are chronic progressive cholestatic diseases with unknown pathological mechanisms, which may ultimately lead to end-stage diseases such as cirrhosis, liver failure, and cholangiocarcinoma (Galoosian, A., et al. (2020). "Clinical Updates in Primary Biliary Cholangitis: Trends, Epidemiology, Diagnostics, and New Therapeutic Approaches." J Clin Transl Hepatol 8(1): 49-60.). PBC occurs mostly in women, with women accounting for as high as 90% of cases; it is an autoimmune disease in which the body's immune system attacks the liver, primarily damaging the small ducts in the liver, leading to itching, fatigue, and metabolic problems in the body. In advanced stages, PBC can cause cirrhosis (end-stage liver disease or stage IV scarring) and liver failure (Horwich, B. H. and H. Han (2021). "Diagnosis and Treatment of Primary Biliary Cholangitis: A Patient-Friendly Summary of the 2018 AASLD Practice Guidance." 18(5): 255-259.). PSC has a much lower incidence compared to PBC, falling into the category of rare diseases; 80% of PSC patients also suffer from inflammatory bowel disease (Mago, S. and G. Y. Wu (2020). "Primary Sclerosing Cholangitis and Primary Biliary Cirrhosis Overlap Syndrome: A Review." J Clin Transl Hepatol 8(3): 336-346.). Moreover, as a chronic progressive disease with an unknown pathogenesis, PSC involves damage to both intrahepatic and extrahepatic bile ducts, thereby inducing a series of gastrointestinal disease symptoms and increasing the incidence of cholangiocarcinoma and colorectal cancer. In terms of treatment, for PBC, the only clinically approved drugs are ursodeoxycholic acid (UDCA) and obeticholic acid (OCA). The former shows inadequate response in clinical patients, while the latter has received a "black box warning" from the FDA for causing dyslipidemia and pruritus. However, for PSC, there are currently no approved drugs for treatment.

Ursodeoxycholic acid (UDCA) is a naturally occurring bile acid, formed by 7β-epimerization of chenodeoxycholic acid (a primary bile acid). It has relatively good hydrophilicity, thus being less toxic than cholic acid or chenodeoxycholic acid. The FDA first approved UDCA in 1987 for dissolution of gallstones and treatment of PBC.

UDCA is currently used as a first-line drug for the treatment of PBC (Gulamhusein, A. F. and G. M. Hirschfield (2020). "Primary biliary cholangitis: pathogenesis and therapeutic opportunities." Nat Rev Gastroenterol Hepatol 17(2): 93-110.), and has demonstrated alleviation effects in various preclinical animal models (Beuers, U., et al. (2015). "New paradigms in the treatment of hepatic cholestasis: from UDCA to FXR, PXR and beyond." J Hepatol 62(1 Suppl): S25-37.; Tang, N., et al. (2018). "The role of ursodeoxycholic acid on cholestatic hepatic fibrosis in infant rats." Mol Med Rep 17(3): 3837-3844.).

Obeticholic acid (OCA) is currently used as a second-line drug for the treatment of PBC. It has demonstrated significant therapeutic effects in mouse models of conditions such as PBC, PSC, non-alcoholic steatohepatitis (NASH), and hepatic fibrosis (Gitto S., et al. (2018). "Guarneri V, Sartini A, et al. The use of obeticholic acid for the management of non-viral liver disease: current clinical practice and future perspectives [J]." Expert Review of Gastroenterology & Hepatology, 2018, 12(2): 165-171.).

Norursodeoxycholic acid (NorUDCA) is an artificially synthesized side-chain-shortened homolog of UDCA, partially resistant to amidation, and capable of undergoing cholehepatic shunting. NorUDCA has been shown to have significant beneficial effects in experimental models of biliary tract injury (Fickert P., et al. (2018). "24-norUrsodeoxycholic acid is superior to ursodeoxycholic acid in the treatment of sclerosing cholangitis in Mdr2 (Abcb4) knockout mice." Gastroenterology. 2006 Feb; 130(2): 465-81.). In addition, it has completed phase 2 clinical trials (NCT01755507).

UDCA has insufficient therapeutic efficacy, with about 25%-50% of patients showing no positive response (Lin-Xiang Huang, et al. (2022). "Incomplete response to ursodeoxycholic acid in primary biliary cholangitis: criteria, epidemiology, and possible mechanisms." Expert Review of Gastroenterology & Hepatology,16:11-12, 1065-1078.). The second-line treatment drug OCA can cause safety problems of pruritus and dyslipidemia; there are currently no drugs available for PSC. There remains a great unmet clinical need for both indications. Since the liver is responsible for the primary function of drug metabolism in the body, higher requirements need to be placed on drug safety in the treatment of hepatobiliary diseases such as PBC or PSC. Therefore, there is an urgent need in the art for a novel therapy for treating liver diseases such as PBC and PSC, which has better therapeutic effects and higher safety compared to existing therapies.

### SUMMARY

The inventors of the present application have addressed the above needs through creative work.

Specifically, the present disclosure relates to the following technical solutions:
In one embodiment, the present disclosure relates to a combination product, which comprises:
(a) a compound of formula (I) or a pharmaceutically acceptable salt, a solvate, a hydrate, or a stereoisomer thereof: 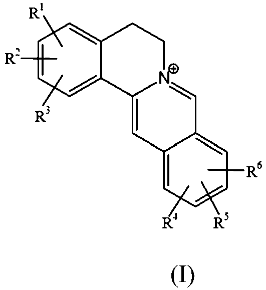
   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N; and
(b) a bile acid or a derivative or an analog thereof, or a pharmaceutically acceptable salt, a solvate, a hydrate, or a stereoisomer thereof.

In some preferred embodiments, the molar ratio of the component (a) to the component (b) is 1:1000-1000:1.

In another embodiment, the present disclosure relates to a pharmaceutical composition, which comprises:
(a) a compound of formula (I) or a pharmaceutically acceptable salt, a solvate, a hydrate, or a stereoisomer thereof: 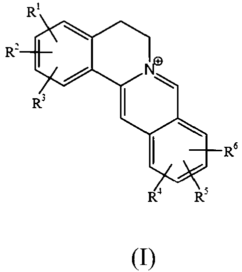 wherein R¹, R², R³, R⁴, R⁵, and R⁶ are as defined herein;
(b) a bile acid or a derivative or an analog thereof, or a pharmaceutically acceptable salt, a solvate, a hydrate, or a stereoisomer thereof; and
(c) a pharmaceutically acceptable carrier, excipient, and/or diluent.

In some preferred embodiments, the molar ratio of the component (a) to the component (b) is 1:1000-1000:1.

In yet another embodiment, the present disclosure relates to an acid-base addition salt of formula (II):

(A⁺)ₘ(B⁻)ₙ(C⁻)ₚ (II)

wherein
(a)A⁺ is a cationic moiety, which is a compound of formula (I): 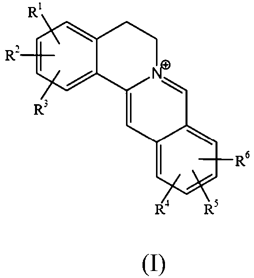 wherein R¹, R², R³, R⁴, R⁵, and R⁶ are as defined herein;
(b) B⁻ is an anionic moiety, which is a bile acid or a derivative or an analog thereof; and
(c) C⁻ is an acid anion;
wherein m, n, and p are each independently an integer selected from 1-6, such that the configuration of the salt reaches charge balance, and when m = n, p is 0.

In still another embodiment, the present disclosure relates to the combination product, the pharmaceutical composition, the acid-base addition salt, or the compound of formula (I) or the pharmaceutically acceptable salt, the solvate, the hydrate, or the stereoisomer thereof according to any one of the preceding embodiments for use in treating, alleviating, and/or preventing a liver disease or inflammatory bowel disease.

In still another embodiment, the present disclosure relates to a method for treating and/or preventing a liver disease or inflammatory bowel disease, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In yet still another embodiment, the present disclosure relates to a kit, which comprises:
the combination product, the acid-base addition salt, the pharmaceutical composition, or the compound of formula (I) or the pharmaceutically acceptable salt, the solvate, the hydrate, or the stereoisomer thereof according to any one of the preceding embodiments, and instructions for use,
wherein the kit is used for treating and/or preventing a liver disease or inflammatory bowel disease.

The details of the present application are set forth in the accompanying description below. Although methods and materials similar or equivalent to those described herein can be used in the practice or testing of the present application, the illustrative methods and materials are now described. Other features, objectives, and advantages of the present application will be apparent from the specification and the claims. In the specification and the appended claims, the singular forms also include their plural forms, unless the context clearly dictates otherwise. Unless otherwise defined, all technical and scientific terms used herein have the same meanings as commonly understood by those of ordinary skill in the art to which the present application belongs. All patents and publications cited in this specification are incorporated herein by reference in their entirety.

The content of all references (including literature references, issued patents, published patent applications, and co-pending patent applications) cited throughout the present application is hereby expressly incorporated by reference in its entirety. Unless otherwise defined, all technical and scientific terms used herein have the meanings commonly known to those of ordinary skill in the art.

### BRIEF DESCRIPTION OF THE DRAWINGS

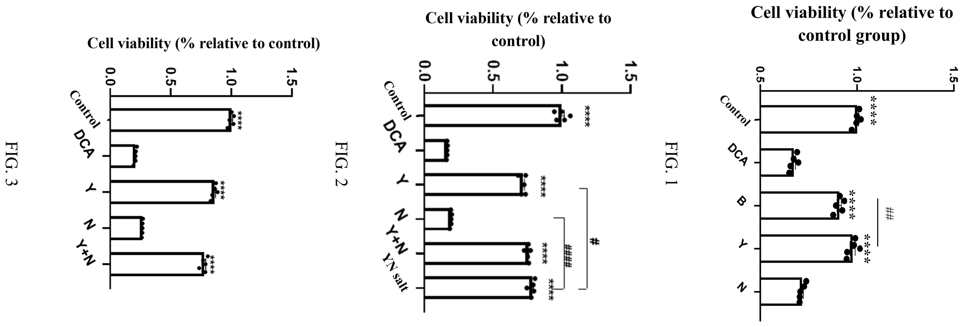

FIG. 1 depicts the effects of compound B, compound Y, and compound N in protecting hepatocytes from deoxycholic acid (DCA)-induced cell damage. In the experiment, the concentrations of compound B and compound Y were 100 µM, and the concentration of compound N was 50 µM. Data are presented as mean ± S.E.M. **** P < 0.0001, vs. DCA, as analyzed by one-way ANOVA in Graphpad Prism 8; ##P < 0.01, as analyzed by unpaired t-test in Graphpad Prism 8.
FIG. 2 depicts the effects of compound Y, compound N, a compound combination Y + N, and a YN salt in protecting hepatocytes from deoxycholic acid (DCA)-induced cell damage. In the experiment, the concentration of the YN salt was 50 µM, which corresponded to proportionally equivalent concentrations of 50 µM for compound Y, 50 µM for compound N, and 50 µM + 50 µM for the compound combination Y + N. Data are presented as mean ± S.E.M. **** P < 0.0001, vs. DCA, as analyzed by one-way ANOVA in Graphpad Prism 8; ^{####}P < 0.0001, ^{#}P < 0.05, vs. YN salt, as analyzed by unpaired t-test.
FIG. 3 depicts the effects of compound Y, compound N, and the compound combination Y + N in protecting hepatocytes from deoxycholic acid (DCA)-induced cell damage. In the experiment, the concentration of compound Y was 100 µM, the concentration of compound N was 50 µM, and the concentration of the compound combination Y + N was 100 µM + 50 µM. Data are presented as mean ± S.E.M. **** P < 0.0001, vs. DCA, as analyzed by one-way ANOVA in Graphpad Prism 8.

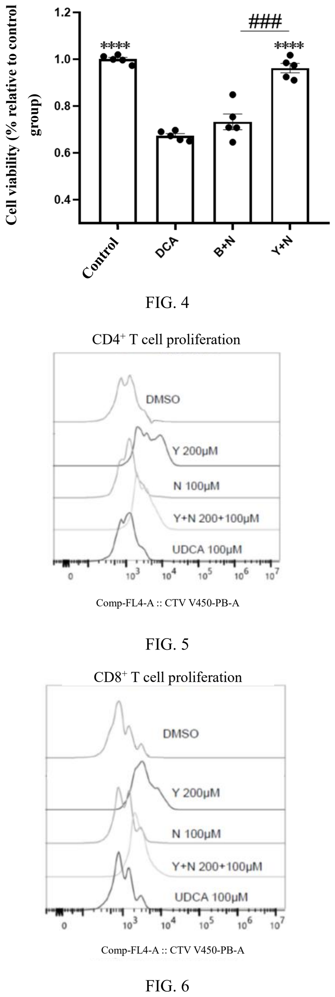

FIG. 4 depicts the effects of a compound combination B + N and the compound combination Y + N in protecting hepatocytes from deoxycholic acid (DCA)-induced cell damage. In the experiment, the concentration of the compound combination Y + N was 100 µM + 50 µM, and the concentration of the compound combination B + N was 100 µM + 50 µM. Data are presented as mean ± S.E.M. **** P < 0.0001, vs. DCA, as analyzed by one-way ANOVA in Graphpad Prism 8; ###P < 0.001, as analyzed by unpaired t-test in Graphpad Prism 8.
FIG. 5 depicts the effects of compound Y, compound N, the compound combination Y + N, and compound UDCA (U) on the proliferation of CD4-positive T cells.In the experiment, the concentration of compound Y was 200 µM, the concentration of compound N was 100 µM, the concentration of the compound combination Y + N was 200 µM + 100 µM, and the concentration of compound U was 100 µM.
FIG. 6 depicts the effects of compound Y, compound N, the compound combination Y + N, and compound UDCA (U) on the proliferation of CD8-positive T cells. In the experiment, the concentration of compound Y was 200 µM, the concentration of compound N was 100 µM, the concentration of the compound combination Y + N was 200 µM + 100 µM, and the concentration of compound U was 100 µM.

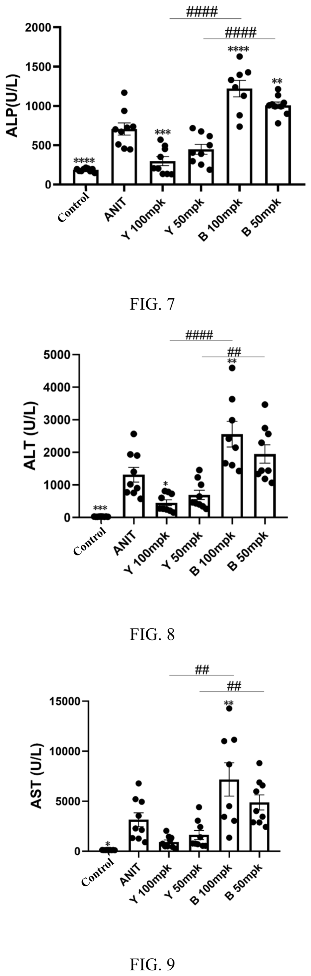

FIG. 7 depicts the effects of different doses of compound Y and compound B on the endpoint alkaline phosphatase (ALP) levels in mice subjected to intragastric administration of α-naphthylisothiocyanate (ANIT). In the experiment, the doses of compound Y were 100 mg/kg and 50 mg/kg, and the doses of compound B were 100 mg/kg and 50 mg/kg. Data are presented as mean ± S.E.M. **** p < 0.0001, ***p < 0.001, **p < 0.01, vs. ANIT, as analyzed by one-way ANOVA in Graphpad Prism 8; ####p < 0.0001, as analyzed by unpaired t-test in Graphpad Prism 8.
FIG. 8 depicts the effects of different doses of compound Y and compound B on the endpoint alanine aminotransferase (ALT) levels in mice subjected to intragastric administration of α-naphthylisothiocyanate (ANIT). In the experiment, the doses of compound Y were 100 mg/kg and 50 mg/kg, and the doses of compound B were 100 mg/kg and 50 mg/kg. Data are presented as mean ± S.E.M. *** p < 0.001, **p < 0.01, *p < 0.05, vs. ANIT, as analyzed by one-way ANOVA in Graphpad Prism 8; ####p < 0.0001, ##p < 0.01, as analyzed by unpaired t-test in Graphpad Prism 8.
FIG. 9 depicts the effects of different doses of compound Y and compound B on the endpoint aspartate aminotransferase (AST) levels in mice subjected to intragastric administration of α-naphthylisothiocyanate (ANIT). In the experiment, the doses of compound Y were 100 mg/kg and 50 mg/kg, and the doses of compound B were 100 mg/kg and 50 mg/kg. Data are presented as mean ± S.E.M. ** p < 0.01, *p < 0.05, vs. ANIT, as analyzed by one-way ANOVA in Graphpad Prism 8; ##p < 0.01, as analyzed by unpaired t-test in Graphpad Prism 8.

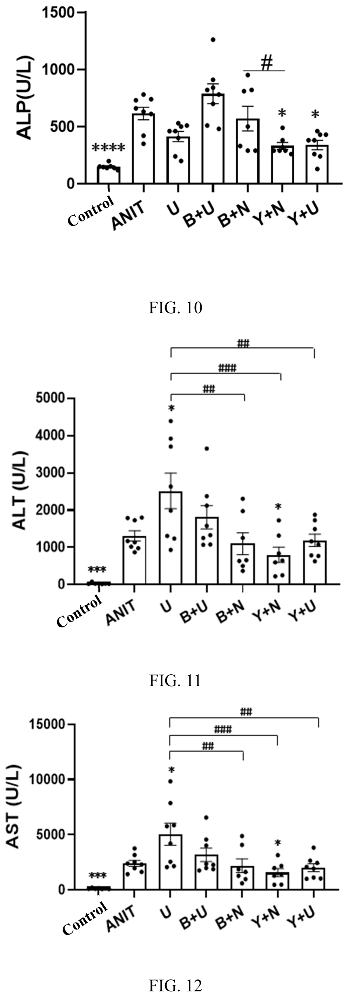

FIG. 10 depicts the effects of the compound combinations (B + U, Y + U, B + N, and Y + N) and the positive control UDCA (U) on the endpoint serum alkaline phosphatase (ALP) levels in mice subjected to intragastric administration of α-naphthylisothiocyanate (ANIT). In the experiment, when the compounds were administered in combination, the dose of B + U was 100 mg/kg (B) + 50 mg/kg (U), the dose of B + N was 100 mg/kg (B) + 50 mg/kg (N), the dose of Y + U was 100 mg/kg (Y) + 50 mg/kg (U), and the dose of Y + N was 100 mg/kg (Y) + 50 mg/kg (N); the dose of UDCA (U) as the positive control was 100 mg/kg. Data are presented as mean ± S.E.M. **** p < 0.0001, *p < 0.05, vs. ANIT, as analyzed by one-way ANOVA in Graphpad Prism 8; ^{#}P < 0.05, compound combination B + N vs. compound combination Y + N, as analyzed by unpaired t-test in Graphpad Prism 8.
FIG. 11 depicts the effects of the compound combinations (B + U, Y + U, B + N, and Y + N) and the positive control UDCA (U) on the endpoint serum alanine transaminase (ALT) levels in mice subjected to intragastric administration of α-naphthylisothiocyanate (ANIT). In the experiment, when the compounds were administered in combination, the dose of B + U was 100 mg/kg (B) + 50 mg/kg (U), the dose of B + N was 100 mg/kg (B) + 50 mg/kg (N), the dose of Y + U was 100 mg/kg (Y) + 50 mg/kg (U), and the dose of Y + N was 100 mg/kg (Y) + 50 mg/kg (N); the dose of UDCA (U) as the positive control was 100 mg/kg. Data are presented as mean ± S.E.M. **** p < 0.0001, *p < 0.05, vs. ANIT; ###p < 0.001, ##p < 0.01, vs. U, as analyzed by one-way ANOVA in Graphpad Prism 8.
FIG. 12 depicts the effects of the compound combinations (B + U, Y + U, B + N, and Y + N) and the positive control UDCA (U) on the endpoint aspartate aminotransferase (AST) levels in mice subjected to intragastric administration of α-naphthylisothiocyanate (ANIT). In the experiment, when the compounds were administered in combination, the dose of B + U was 100 mg/kg (B) + 50 mg/kg (U), the dose of B + N was 100 mg/kg (B) + 50 mg/kg (N), the dose of Y + U was 100 mg/kg (Y) + 50 mg/kg (U), and the dose of Y + N was 100 mg/kg (Y) + 50 mg/kg (N); the dose of UDCA (U) as the positive control was 100 mg/kg. Data are presented as mean ± S.E.M. **** p < 0.0001, *p < 0.05, vs. ANIT; ###p < 0.001, ##p < 0.01, vs. U, as analyzed by one-way ANOVA in Graphpad Prism 8.

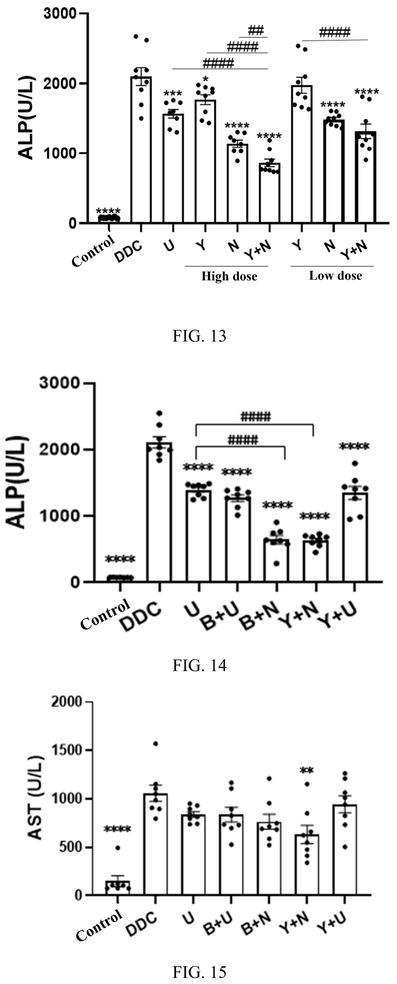

FIG. 13 depicts the effects of compound Y, compound N, the compound combination Y + N, and the positive control UDCA (U) on the serum alkaline phosphatase (ALP) levels in mice fed with feed containing 0.1% diethyl 1,4-dihydro-2,4,6-trimethyl-3,5-pyridinedicarboxylate (DDC model).In the experiment, the dose of high-dose compound Y was 100 mg/kg, the dose of compound N was 56.5 mg/kg, and the dose of the compound combination Y + N was 100 mg/kg (Y) + 56.5 mg/kg (N); the concentration of low-dose compound Y was 60 mg/kg, and the concentration of compound N was 34 mg/kg, and the dose of the compound combination Y + N was 60 mg/kg (Y) + 34 mg/kg (N); the dose of UDCA was 100 mg/kg. Data are presented as mean ± S.E.M. **** P < 0.0001, ***P < 0.001, *P < 0.05, vs. DDC, as analyzed by one-way ANOVA in Graphpad Prism 8; ^{####}P < 0.0001, ^{##}P < 0.01, vs. compound combination Y + N, as analyzed by one-way ANOVA in Graphpad Prism 8.
FIG. 14 depicts the effects of the compound combinations (B + U, Y + U, B + N, and Y + N) and the positive control UDCA (U) on the endpoint serum alkaline phosphatase (ALP) levels in mice fed with feed containing 0.1% diethyl 1,4-dihydro-2,4,6-trimethyl-3,5-pyridinedicarboxylate (DDC model). When the compounds were administered in combination, the dose of each compound was 100 mg/kg; the concentration of UDCA (U) as the positive control was 100 mg/kg. Data are presented as mean ± S.E.M. **** p < 0.0001, **p < 0.01, *p < 0.05, vs. DDC; ####p < 0.0001, vs. U, as analyzed by one-way ANOVA in Graphpad Prism 8.
FIG. 15 depicts the effects of the compound combinations (B + U, Y + U, B + N, and Y + N) and the positive control UDCA (U) on the endpoint serum aspartate aminotransferase (AST) levels in mice fed with feed containing 0.1% diethyl 1,4-dihydro-2,4,6-trimethyl-3,5-pyridinedicarboxylate (DDC model). When the compounds were administered in combination, the dose of each compound was 100 mg/kg; the concentration of UDCA (U) as the positive control was 100 mg/kg. Data are presented as mean ± S.E.M. **** p < 0.0001, **p < 0.01, *p < 0.05, vs. DDC, as analyzed by one-way ANOVA in Graphpad Prism 8.

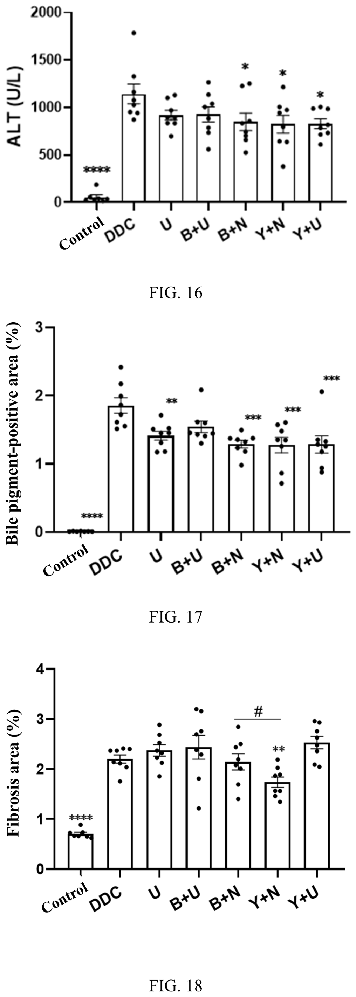

FIG. 16 depicts the effects of the compound combinations (B + U, Y + U, B + N, and Y + N) and the positive control UDCA (U) on the endpoint serum alanine transaminase (ALT) levels in mice fed with feed containing 0.1% diethyl 1,4-dihydro-2,4,6-trimethyl-3,5-pyridinedicarboxylate (DDC model). When the compounds were administered in combination, the dose of each compound was 100 mg/kg; the concentration of UDCA (U) as the positive control was 100 mg/kg. Data are presented as mean ± S.E.M. **** p < 0.0001, **p < 0.01, *p < 0.05, vs. DDC, as analyzed by one-way ANOVA in Graphpad Prism 8.
FIG. 17 depicts the effects of the compound combinations (B + U, Y + U, B + N, and Y + N) and the positive control UDCA (U) on the endpoint hepatic bile pigment deposition area in mice fed with feed containing 0.1% diethyl 1,4-dihydro-2,4,6-trimethyl-3,5-pyridinedicarboxylate (DDC model). When the compounds were administered in combination, the dose of each compound was 100 mg/kg; the concentration of UDCA (U) as the positive control was 100 mg/kg. Data are presented as mean ± S.E.M. **** p < 0.0001, ***p < 0.001, **p < 0.01, vs. DDC, as analyzed by one-way ANOVA in Graphpad Prism 8.
FIG. 18 depicts the effects of the compound combinations (B + U, Y + U, B + N, and Y + N) and the positive control UDCA (U) on the endpoint hepatic fibrosis area in mice fed with feed containing 0.1% diethyl 1,4-dihydro-2,4,6-trimethyl-3,5-pyridinedicarboxylate (DDC model). When the compounds were administered in combination, the dose of each compound was 100 mg/kg; the concentration of UDCA (U) as the positive control was 100 mg/kg. Data are presented as mean ± S.E.M. **** p < 0.0001, **p < 0.01, *p < 0.05, vs. DDC, as analyzed by one-way ANOVA in Graphpad Prism 8; ^{#}P < 0.05, vs. compound combination Y + N, as analyzed by unpaired t-test in Graphpad Prism 8.

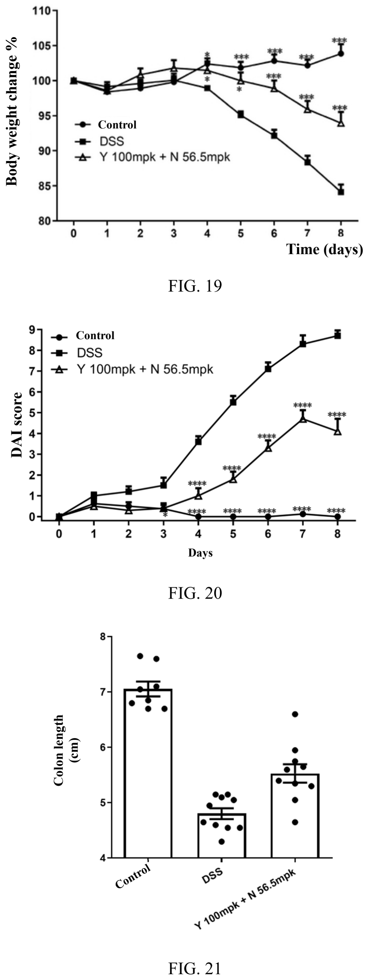

FIG. 19 depicts the effect of the compound combination Y + N on the body weight of mice at the endpoint of an enteritis model induced by adding dextran sulfate sodium (DSS) to drinking water. In the experiment, the dose of the compound combination Y + N was 100 mg/kg + 56.5 mg/kg (the molar ratio of Y:N was about 2:1). Data are presented as mean ± S.E.M. *** p < 0.001, **p < 0.01, *p < 0.05, vs. DSS, as analyzed by one-way ANOVA in Graphpad Prism 8.
FIG. 20 depicts the effect of the compound combination Y + N on the DAI scores of mice at the endpoint of the enteritis model induced by adding dextran sulfate sodium (DSS) to drinking water. In the experiment, the dose of the compound combination Y + N was 100 mg/kg + 56.5 mg/kg (the molar ratio of Y:N was about 2:1). Data are presented as mean ± S.E.M. **** p < 0.0001, *p < 0.05, vs. DSS, as analyzed by one-way ANOVA in Graphpad Prism 8.
FIG. 21 depicts the effect of the compound combination Y + N on the colon length of mice at the endpoint of the enteritis model induced by adding dextran sulfate sodium (DSS) to drinking water. In the experiment, the dose of the compound combination Y + N was 100 mg/kg + 56.5 mg/kg (the molar ratio of Y:N was about 2:1). Data are presented as mean ± S.E.M. **** p < 0.0001, **p < 0.01, vs. DSS, as analyzed by one-way ANOVA in Graphpad Prism 8.

### DETAILED DESCRIPTION

### Definitions

In the present disclosure, unless otherwise stated, the scientific and technical terms used herein have the meanings generally understood by those skilled in the art. Moreover, the terms and laboratory procedures related to protein and nucleic acid chemistry, molecular biology, cell and tissue culture, microbiology, and immunology used herein are the terms and conventional procedures widely used in the corresponding fields. Meanwhile, in order to better understand the present disclosure, the definitions and explanations of the related terms are provided below.

As used herein, the term "halogen" refers to fluorine, chlorine, bromine, or iodine.

As used herein, the term "alkyl" refers to a saturated aliphatic hydrocarbon group that is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. Lower alkyl groups containing 1 to 6 carbon atoms are more preferred, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted, and when it is substituted, the substituent may be attached at any accessible point of attachment, and the substituent is preferably one or more groups independently selected from alkyl, alkenyl, alkynyl, alkoxy, alkylthio, alkylamino, halogen, sulfhydryl, hydroxyl, nitro, amino, cycloalkyl, heterocycloalkyl, aryl, heteroaryl, cycloalkoxy, heterocycloalkoxy, cycloalkylthio, heterocycloalkylthio, and oxo.

As used herein, the term "alkoxy" refers to -O-(alkyl) and -O-(cycloalkyl), wherein the alkyl or cycloalkyl is as defined above. Non-limiting examples of alkoxy include: methoxy, ethoxy, propoxy, butoxy, cyclopropyloxy, cyclobutyloxy, cyclopentyloxy, and cyclohexyloxy. Alkoxy may be optionally substituted or unsubstituted, and when it is substituted, the substituent is preferably one or more groups independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, and heterocycloalkylthio.

As used herein, the terms "halo" and "halogen" refer to fluorine, chlorine, bromine, and iodine.

As used herein, the term "haloalkyl", by itself or as part of another substituent, refers to an alkyl group in which some or all of the hydrogen atoms are replaced by halogen atoms. For alkyl groups, the haloalkyl may have any suitable number of carbon atoms, e.g., C1-6. For example, the haloalkyl includes trifluoromethyl, fluoromethyl, etc. In some contexts, the term "perfluoro" can be used to define a compound or group in which all hydrogen atoms are replaced by fluorine atoms. For example, perfluoromethyl refers to 1,1,1-trifluoromethyl.

As used herein, the term "hydroxyl" refers to an -OH moiety.

The present disclosure also comprises various deuterated forms of formula (I). Each available hydrogen atom attached to a carbon atom may be independently replaced by a deuterium atom. Those skilled in the art are able to synthesize the deuterated forms of formula (I) with reference to the relevant literature. Commercially available deuterated starting materials can be used in preparing the deuterated forms of formula (I), or they can be synthesized using conventional techniques with deuterated reagents. Non-limiting examples of deuterated reagents include: deuterated borane, tri-deuterated borane in tetrahydrofuran, deuterated lithium aluminum hydride, deuterated iodoethane, deuterated iodomethane, etc.

"Pharmaceutically acceptable" means that the component is compatible with the other components of the formulation and is harmless to the recipient.

As used herein, the term "pharmaceutically acceptable salt", when used in this context, refers to a pharmaceutically acceptable organic or inorganic salt of the compound. Exemplary salts include, but are not limited to, sulfate, citrate, acetate, oxalate, hydrochloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)). The pharmaceutically acceptable salt may involve the inclusion of another molecule, such as an acetate ion, a succinate ion, or another counterions. The counterion may be any organic or inorganic moiety that stabilizes the charge of the parent compound. In addition, the pharmaceutically acceptable salt may have more than one charged atom in the structure. In cases where multiple charged atoms are components of a pharmaceutically acceptable salt, multiple counterions can be present. Therefore, the pharmaceutically acceptable salt may have one or more charged atoms and/or one or more counterions.

As used herein, the term "acid anion" refers to an anion produced upon ionization of an acid. Common acid anions include chloride ion, bromide ion, iodide ion, carbonate ion, bicarbonate ion, sulfate ion, phosphate ion, hydrogen phosphate ion, dihydrogen phosphate ion, formate ion, acetate ion, etc. The valence of the acid anion may be -1, -2, -3, -4, etc.

As used herein, the term "pharmaceutically acceptable carrier" refers to a conventional non-toxic solid, semi-solid, or liquid filler, diluent, encapsulating material, formulation auxiliary, or carrier used in the art together with a therapeutic agent, collectively constituting a "pharmaceutical composition" for administration to an individual. Pharmaceutically acceptable carriers are non-toxic to recipients at the doses and concentrations employed and are compatible with the other ingredients of the formulation. Pharmaceutically acceptable carriers are suitable for the formulation employed.

As used herein, the term "pharmaceutically acceptable excipient" refers to a substance that facilitates the administration of an active agent to an individual. Useful pharmaceutical excipients include, but are not limited to, binders, fillers, disintegrants, lubricants, glidants, coating agents, sweetening agents, flavoring agents, and coloring agents.

Depending on the location and nature of the various substituents desired, the compounds of the present disclosure may contain one or more asymmetric centers. Asymmetric carbon atoms may exist in the (R)- and/or (S)-configuration, resulting in racemic mixtures in the case of a single asymmetric center, and in diastereomeric mixtures in the case of multiple asymmetric centers. In some cases, asymmetry may also exist due to hindered rotation about a particular bond, such as a central bond connecting two substituted aromatic rings of a particular compound. Substituents on rings may also exist in cis or trans forms. It is intended that all such configurations (including enantiomers and diastereomers) are included within the scope of the present disclosure. Preferred compounds are those that produce more of the desired biological activities. Separated, pure, or partially purified isomers and stereoisomers, or racemic or diastereomeric mixtures of the compounds of the present disclosure are all encompassed within the scope of the present disclosure. Purification and separation of such substances can be accomplished by standard techniques known in the art.

Tautomers, sometimes referred to as proton-shift tautomers, are two or more compounds that are associated by the migration of a hydrogen atom, accompanied by the switching of one or more single bonds and one or more adjacent double bonds. The compounds of the present disclosure may exist in one or more tautomeric forms.

The present disclosure also includes all suitable isotopic variants of the compounds of the present disclosure. Isotopic variants of the compounds of the present disclosure are defined as: compounds of the present disclosure in which at least one atom is replaced by an atom with the same atomic number but an atomic mass different from the atomic mass usually or predominantly found in nature. Examples of isotopes that can be incorporated into the compounds of the present disclosure include isotopes of hydrogen, carbon, nitrogen, oxygen, phosphorus, sulfur, fluorine, chlorine, bromine, and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I, and ¹³¹I. Certain isotopic variants of the compounds of the present disclosure, for example, those variants into which one or more radioactive isotopes (e.g., ³H or ¹⁴C) are incorporated, can be used in drug and/or substrate tissue distribution studies. Tritium and carbon-14 (i.e., ¹⁴C) isotopes are particularly preferred due to their ease of preparation and detectability. Furthermore, substitution with isotopes such as deuterium may afford certain therapeutic advantages resulting from better metabolic stability, for example, increased *in vivo* half-life or reduced dose requirements, and hence may be preferred in some circumstances. Isotopic variants of the compounds of the present disclosure can generally be prepared by employing conventional methods known to those skilled in the art, for example, by employing the illustrative methods or the preparation methods described in the examples below using appropriate isotopic variants of suitable reagents.

The present disclosure includes all possible stereoisomers of the compounds of the present disclosure as single stereoisomers or any mixture of said stereoisomers in any ratio. Separation of single stereoisomers, such as single enantiomers or single diastereomers, of the compounds of the present disclosure may be achieved by any suitable method in the prior art, such as chromatography, particularly, for example, chiral chromatography.

The present disclosure includes all possible tautomers of the compounds of the present disclosure, either as single tautomers or as any mixture of said tautomers in any ratio.

As used herein, the term "solvate" refers to a pharmaceutically acceptable solvate formed by the compound of the present disclosure with one or more solvent molecules; non-limiting examples of the solvent molecules include water, ethanol, acetonitrile, isopropanol, DMSO, ethyl acetate, etc. Hydrates are a specific form of solvates in which the solvent is water.

As used herein, the terms "effective amount" and "therapeutically effective amount" refer to a dose of a compound, such as the compound, the compound combination or the salt form of the present disclosure, that produces the therapeutic effect for which it is administered. The exact dose will depend on the purpose of the treatment and will be determined by those skilled in the art using known techniques (see, e.g., Lieberman, Pharmaceutical Dosage Forms (vols. 1-3, 1992); Lloyd, The Art, Science and Technology of Pharmaceutical Compounding (1999); Pickar, Dosage Calculations (1999); Goodman & Gilman's The Pharmacological Basis of Therapeutics, 11th Edition, 2006, Brunton, Ed., McGraw-Hill; and Remington: The Science and Practice of Pharmacy, 21st Edition, 2005, Hendrickson, Ed., Lippincott, Williams & Wilkins).

As used herein, "treatment" or "treating" is an approach for obtaining beneficial or desired results (including clinical results). For the purposes of the present application, beneficial or desired clinical results include, but are not limited to, one or more of the following: alleviating one or more symptoms resulting from the disease, reducing the severity of the disease, stabilizing the disease (e.g., preventing or delaying the worsening of the disease), preventing or delaying the spread (e.g., metastasis) of the disease, preventing or delaying the recurrence of the disease, delaying or slowing the progression of the disease, ameliorating the disease state, providing a remission (partial or complete) of the disease, decreasing the dose of one or more other medications required to treat the disease, delaying the progression of the disease, increasing or improving the quality of life, increasing weight gain, and/or extending survival. "Treatment" also encompasses reducing the pathological consequences (such as, for example, tumor volume) of cancer. The methods of the present application contemplate any one or more of these aspects of treatment.

As used herein, "prevention" or "preventing" includes providing prophylaxis against the occurrence or recurrence of a disease in an individual who may be predisposed to the disease but has not yet been diagnosed with the disease.

The terms "subject", "individual", and "patient" are used interchangeably herein to refer to a mammal, including, but not limited to, human, bovine, horse, feline, canine, rodent, or primate. In some embodiments, the individual is a human.

An "effective amount" of a pharmaceutical agent refers to an amount effective, at the required dose and over the required period of time, to achieve the desired therapeutic or prophylactic result. The specific dose may vary depending on one or more of the following: the particular pharmaceutical agent chosen, the dosing regimen to be followed, whether it is administered in combination with other compounds, the timing of administration, the tissue to be imaged, and the physical delivery system in which it is carried.

As used herein, the term "synergy" or "synergistic" is used to refer to a result of a combination of two compounds, components, or targeting agents that is greater than the sum of the individual pharmaceutical agents together. The term "synergy" or "synergistic" refers to an amelioration in a disease condition or disorder being treated as compared to the use of each compound, component, or targeting agent alone. This amelioration in the disease condition or disorder being treated is a "synergistic effect". A "synergistic amount" is an amount of a combination of two compounds, components, or targeting agents that results in a synergistic effect ("synergistic" is as defined herein).

The determination of the synergistic interaction between one or two components, the optimal range of the effect, and the absolute dose range of each component for the effect can be definitively measured by administering the components within different w/w ratio ranges and at different doses to patients in need of treatment. However, the observation of synergy in *in vitro* or *in vivo* models can be predictive of the effects in humans and other species, as well as in existing *in vitro* or *in vivo* models as described herein, for measuring synergistic effects. Furthermore, the results of such studies can also be used to predict the required effective dose and plasma concentration ratio ranges, as well as absolute doses and plasma concentrations, in humans and other species by applying pharmacokinetic/pharmacodynamic methods.

It should be understood that the embodiments of the application described herein include "consisting of..." and/or "consisting essentially of...".

Reference herein to an "about" value or parameter includes (and describes) variations that are directed to that value or parameter itself. For example, a description referring to "about X" includes a description of "X".

As used herein, the term "about X-Y" has the same meaning as "about X to about Y".

As used herein, the term "about", when used to modify the quantity of an ingredient or reactant of the present disclosure, refers to variations in the numerical amount that may occur, for example, from typical measurements and liquid handling procedures used in preparing concentrates or working solutions; from accidental errors in these procedures; from differences in the manufacture, source, or purity of the ingredients used in preparing the composition or implementing the method; etc. The term "about" also encompasses amounts that vary due to different equilibrium conditions relative to the composition resulting from a particular starting mixture. Whether modified by the term "about" or not, the claims include equivalents to the amounts. In one embodiment, the term "about" means a variation within 10% of the reported numerical value, preferably a variation within 5% of the reported numerical value.

It should be understood by those skilled in the art that when a numerical value is recited in a claim, whether it is prefixed with "about" or not, the actual value of the numerical value may vary above or below the recited value by 10% (±10%), preferably by 5% (±5%).

When used herein and in the appended claims, the singular forms "a", "an", "or", and "the" include plural referents, unless the context clearly dictates otherwise.

### Description of Embodiments

### Combination Product

In some aspects, the present disclosure provides a combination product, which comprises:
(a) a compound of formula (I) or a pharmaceutically acceptable salt, a solvate, a hydrate, or a stereoisomer thereof: 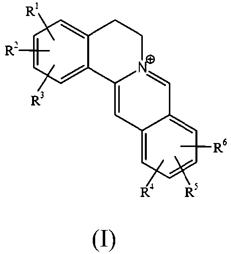
   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N; and
(b) a bile acid or a derivative or an analog thereof, or a pharmaceutically acceptable salt, a solvate, a hydrate, or a stereoisomer thereof.

In a further embodiment, the molar ratio of the component (a) to the component (b) is 1:1000-1000:1.

In a further embodiment, R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms. In a yet further embodiment, one or two of R¹, R², and R³ are hydrogen; or any two adjacent ones of R¹, R², and R³, together with the carbon atoms to which they are attached, form a 5-membered heterocyclic ring containing one or more oxygen atoms. In a still further embodiment, one of R¹, R², and R³ is hydrogen; or any two adjacent ones of R¹, R², and R³, together with the carbon atoms to which they are attached, form a 5-membered heterocyclic ring containing two oxygen atoms.

In a further embodiment, R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms. In a yet further embodiment, one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms. In a still further embodiment, one of R⁴, R⁵, and R⁶ is hydrogen; or any two adjacent ones of R⁴, R⁵, and R⁶, together with the carbon atoms to which they are attached, form a 5-membered heterocyclic ring containing two oxygen atoms.

In a further embodiment, the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate. Preferably, the pharmaceutically acceptable salt of the compound of formula (I) is selected from a maleate and a hydrochloride. More preferably, the pharmaceutically acceptable salt of the compound of formula (I) is a hydrochloride.

In a further embodiment, the compound of formula (I) is selected from: 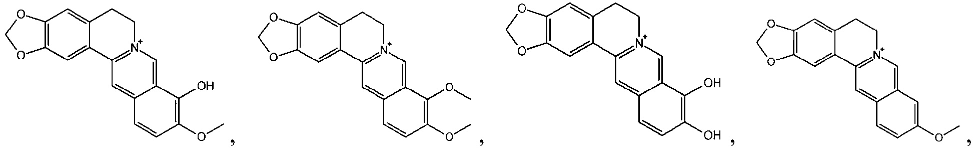 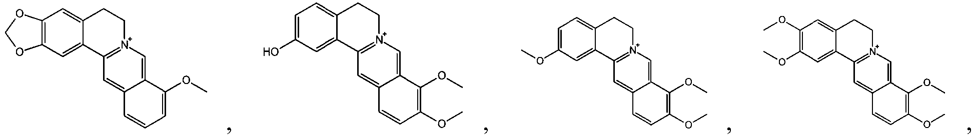 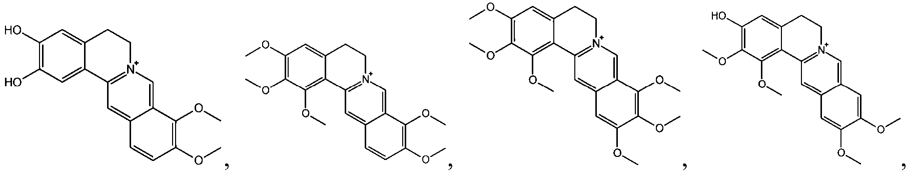 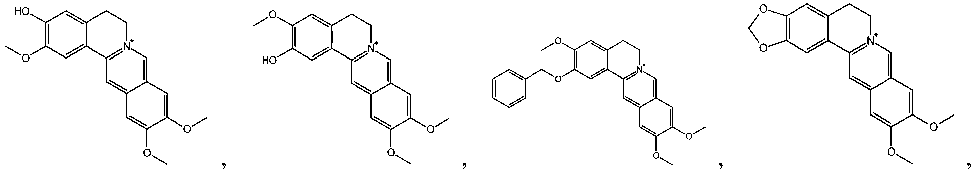 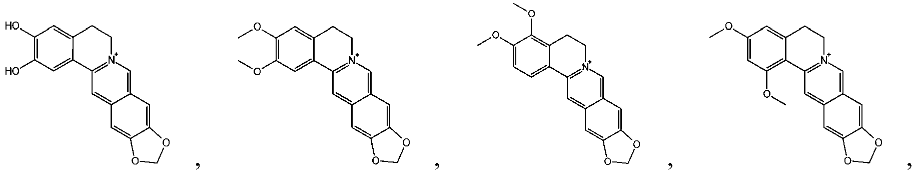 and

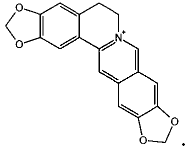

In a further embodiment, the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, tauroursodeoxycholic acid, etc.

In a further embodiment, the molar ratio of the component (a) to the component (b) is 1:100-100:1. Preferably, the molar ratio of the component (a) to the component (b) is 1:90-90:1, 1:80-80:1, 1:70-70:1, 1:60-60:1, 1:50-50:1, 1:40-40:1, 1:30-30:1, 1:20-20:1, or 1:10-10:1. More preferably, the molar ratio of the component (a) to the component (b) is 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or 10:1, or a numerical value between any two of these ratios. In some embodiments, the molar ratio of the component (a) to the component (b) is 1:5-5:1, preferably 1:2-2:1, and more preferably 1:1-2:1. In some embodiments, the molar ratio of the component (a) to the component (b) is 1:1 or 2:1.

In a further embodiment, the combination product is used for treating and/or preventing a liver disease or inflammatory bowel disease, preferably a chronic cholestatic liver disease. Preferably, the liver disease is selected from a cholestatic liver disease (chronic cholestatic liver disease), primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma. More preferably, the liver disease is primary sclerosing cholangitis (PSC) or primary biliary cirrhosis (PBC). Even more preferably, the primary sclerosing cholangitis (PSC) or primary biliary cirrhosis (PBC) is accompanied by inflammatory bowel disease as a complication. Preferably, the inflammatory bowel disease is a complication arising from primary sclerosing cholangitis (PSC) and/or primary biliary cirrhosis (PBC).

In a further embodiment, the component (a) and the component (b) exhibit a synergistic effect in alleviating deoxycholic acid-induced hepatocyte damage.

### Pharmaceutical Composition

In some aspects, the present disclosure provides a pharmaceutical composition, which comprises:
(a) a compound of formula (I) or a pharmaceutically acceptable salt, a solvate, a hydrate, or a stereoisomer thereof: 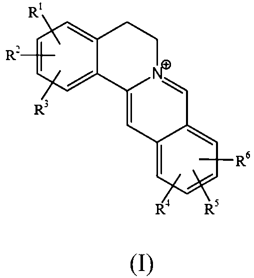
   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N;
(b) a bile acid or a derivative or an analog thereof, or a pharmaceutically acceptable salt, a solvate, a hydrate, or a stereoisomer thereof; and
(c) a pharmaceutically acceptable carrier, excipient, and/or diluent.

In a further embodiment, the molar ratio of the component (a) to the component (b) is 1:1000-1000:1.

In a further embodiment, R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms. In a yet further embodiment, one or two of R¹, R², and R³ are hydrogen; or any two adjacent ones of R¹, R², and R³, together with the carbon atoms to which they are attached, form a 5-membered heterocyclic ring containing one or more oxygen atoms. In a still further embodiment, one of R¹, R², and R³ is hydrogen; or any two adjacent ones of R¹, R², and R³, together with the carbon atoms to which they are attached, form a 5-membered heterocyclic ring containing two oxygen atoms.

In a further embodiment, R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms. In a yet further embodiment, one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms. In a still further embodiment, one of R⁴, R⁵, and R⁶ is hydrogen; or any two adjacent ones of R⁴, R⁵, and R⁶, together with the carbon atoms to which they are attached, form a 5-membered heterocyclic ring containing two oxygen atoms.

In a further embodiment, the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate. Preferably, the pharmaceutically acceptable salt of the compound of formula (I) is selected from a maleate and a hydrochloride. More preferably, the pharmaceutically acceptable salt of the compound of formula (I) is a hydrochloride.

In a further embodiment, the compound of formula (I) is selected from: 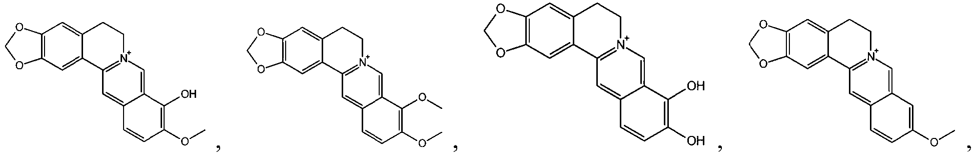 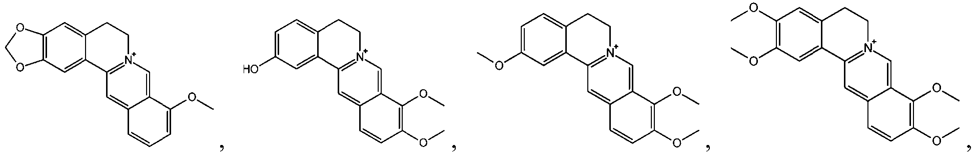 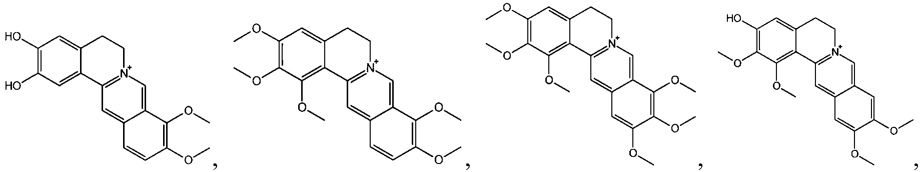 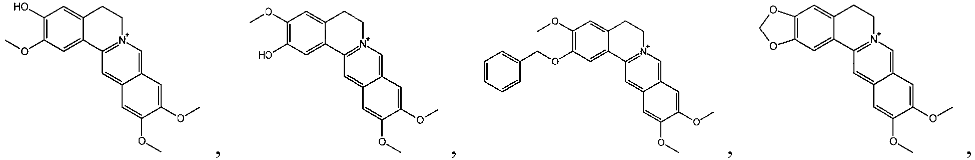 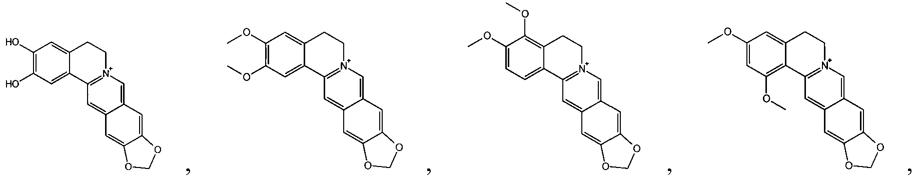 and

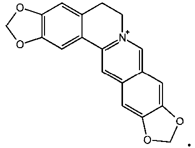

In a further embodiment, the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, tauroursodeoxycholic acid, etc.

In a further embodiment, the molar ratio of the component (a) to the component (b) is 1:100-100:1. Preferably, the molar ratio of the component (a) to the component (b) is 1:90-90:1, 1:80-80:1, 1:70-70:1, 1:60-60:1, 1:50-50:1, 1:40-40:1, 1:30-30:1, 1:20-20:1, or 1:10-10:1. More preferably, the molar ratio of the component (a) to the component (b) is 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or 10:1, or a numerical value between any two of these ratios. In some embodiments, the molar ratio of the component (a) to the component (b) is 1:5-5:1, preferably 1:2-2:1, and more preferably 1:1-2:1. In some embodiments, the molar ratio of the component (a) to the component (b) is 1:1 or 2:1.

In a further embodiment, the pharmaceutical composition is used for treating and/or preventing a liver disease or inflammatory bowel disease, preferably a chronic cholestatic liver disease. Preferably, the liver disease is selected from a cholestatic liver disease (chronic cholestatic liver disease), primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma. More preferably, the liver disease is primary sclerosing cholangitis (PSC) or primary biliary cirrhosis (PBC). Even more preferably, the primary sclerosing cholangitis (PSC) or primary biliary cirrhosis (PBC) is accompanied by inflammatory bowel disease as a complication. Preferably, the inflammatory bowel disease is a complication arising from primary sclerosing cholangitis (PSC) and/or primary biliary cirrhosis (PBC).

In a further embodiment, the component (a) and the component (b) exhibit a synergistic effect in alleviating deoxycholic acid-induced hepatocyte damage.

### Acid-Base Addition Salts

In some aspects, the present disclosure provides an acid-base addition salt of formula (II):

(A⁺)ₘ(B⁻)ₙ(C⁻)ₚ (II)

wherein
(a) A⁺ is a cationic moiety, which is a compound of formula (I): 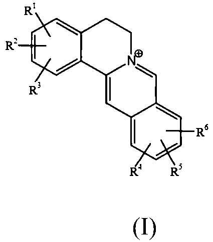
   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N;
(b) B⁻ is an anionic moiety, which is a bile acid or a derivative or an analog thereof; and
(c) C⁻ is an acid anion;
wherein m, n, and p are each independently an integer selected from 1-6, such that the configuration of the salt reaches charge balance, and when m = n, p is 0.

In a further embodiment, R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms. In a yet further embodiment, one or two of R¹, R², and R³ are hydrogen; or any two adjacent ones of R¹, R², and R³, together with the carbon atoms to which they are attached, form a 5-membered heterocyclic ring containing one or more oxygen atoms. In a still further embodiment, one of R¹, R², and R³ is hydrogen; or any two adjacent ones of R¹, R², and R³, together with the carbon atoms to which they are attached, form a 5-membered heterocyclic ring containing two oxygen atoms.

In a further embodiment, R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms. In a yet further embodiment, one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms. In a still further embodiment, one of R⁴, R⁵, and R⁶ is hydrogen; or any two adjacent ones of R⁴, R⁵, and R⁶, together with the carbon atoms to which they are attached, form a 5-membered heterocyclic ring containing two oxygen atoms.

In a further embodiment, the compound of formula (I) is a cationic moiety selected from the group consisting of: 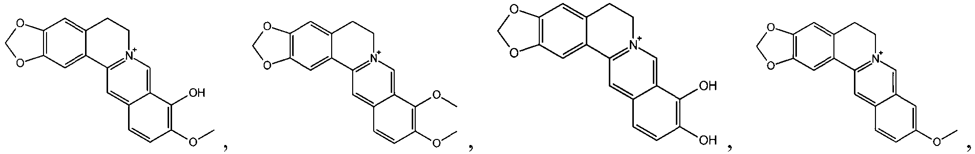 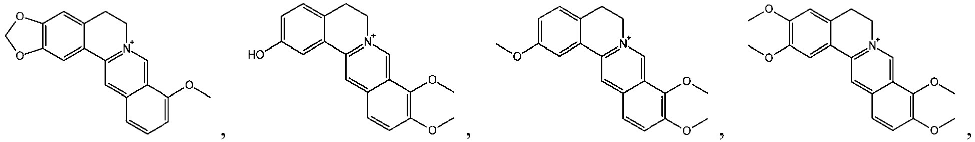 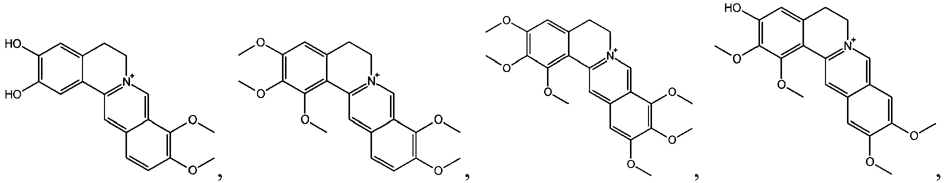 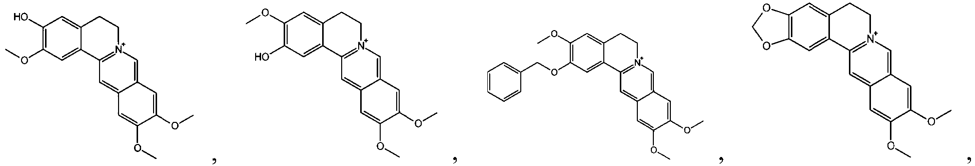 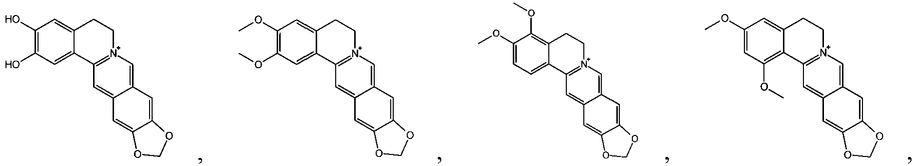 and

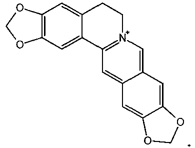

In a further embodiment, the component (b) is an anionic moiety selected from the group consisting of: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, tauroursodeoxycholic acid, etc.

In a further embodiment, C⁻ is a monovalent, divalent, trivalent, or tetravalent acid anion, or a mixture thereof. Preferably, C⁻ is a monovalent, divalent, or trivalent acid anion, or a mixture thereof. More preferably, C⁻ is a monovalent or divalent acid anion, or a mixture thereof. Most preferably, C⁻ is a monovalent acid anion or a mixture thereof.

In a further embodiment, m is 1, n is 1, and p is 0; or m is 2, n is 1, and p is 1.

In a further embodiment, the pharmaceutical composition is used for treating and/or preventing a liver disease or inflammatory bowel disease, preferably a chronic cholestatic liver disease. Preferably, the liver disease is selected from a cholestatic liver disease (chronic cholestatic liver disease), primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma. More preferably, the liver disease is primary sclerosing cholangitis (PSC) or primary biliary cirrhosis (PBC). Even more preferably, the primary sclerosing cholangitis (PSC) or primary biliary cirrhosis (PBC) is accompanied by inflammatory bowel disease as a complication. Preferably, the inflammatory bowel disease is a complication arising from primary sclerosing cholangitis (PSC) and/or primary biliary cirrhosis (PBC).

### Treatment and/or Prevention

In some aspects, the present disclosure provides a method for treating and/or preventing a liver disease. In some aspects, the method for treating and/or preventing the liver disease comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing a cholestatic liver disease, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing primary sclerosing cholangitis (PSC), which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments. In some embodiments, the primary sclerosing cholangitis (PSC) is accompanied by inflammatory bowel disease as a complication.

In some aspects, the present disclosure provides a method for treating and/or preventing primary biliary cirrhosis (PBC), which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments. In some embodiments, the primary biliary cirrhosis (PBC) is accompanied by inflammatory bowel disease as a complication.

In some aspects, the present disclosure provides a method for treating and/or preventing progressive familial intrahepatic cholestasis (particularly progressive familial intrahepatic cholestasis types 1, 2, and 3), which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing cystic fibrosis, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing a cholestatic liver disease, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing drug-induced cholestasis or a non-cholestatic liver disease, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing chronic viral hepatitis (B, C, and D), which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing alcoholic or non-alcoholic steatohepatitis, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing autoimmune hepatitis, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing hemochromatosis, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing Wilson's disease, α-1-antitrypsin deficiency, liver cancer (particularly hepatocellular carcinoma), or cholangiocarcinoma, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing inflammatory bowel disease, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments. Preferably, the inflammatory bowel disease is a complication arising from primary sclerosing cholangitis (PSC) and/or primary biliary cirrhosis (PBC).

In some aspects, the present disclosure provides a method for treating and/or preventing a liver disease or inflammatory bowel disease, which comprises:
regimen 1: administering to a patient in need thereof:
   (a) a therapeutically and/or prophylactically effective amount of a compound of formula (I) or a pharmaceutically acceptable salt, a solvate, a hydrate, or a stereoisomer thereof:

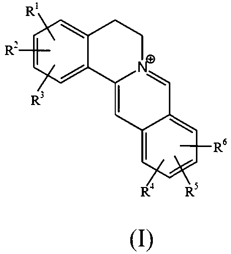

   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N; or
regimen 2: administering to a patient in need thereof the following components simultaneously, concurrently, separately, or sequentially:
   (a) a therapeutically and/or prophylactically effective amount of a compound of formula (I) or a pharmaceutically acceptable salt, a solvate, a hydrate, or a stereoisomer thereof: 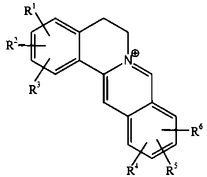 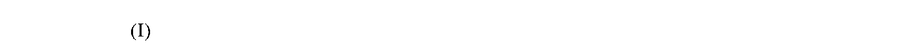
      wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
      or any two adjacent ones of R¹, R², and R³, together with the carbon atoms to which they are attached, form a 4- to 7-membered heterocyclic ring containing one or more heteroatoms selected from O, S, and N;
      or any two adjacent ones of R⁴, R⁵, and R⁶, together with the carbon atoms to which they are attached, form a 4- to 7-membered heterocyclic ring containing one or more heteroatoms selected from O, S, and N; and
   (b) a therapeutically and/or prophylactically effective amount of a bile acid or a derivative or an analog thereof, or a pharmaceutically acceptable salt, a solvate, a hydrate, or a stereoisomer thereof.

In a further embodiment, R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms. In a yet further embodiment, one or two of R¹, R², and R³ are hydrogen; or any two adjacent ones of R¹, R², and R³, together with the carbon atoms to which they are attached, form a 5-membered heterocyclic ring containing one or more oxygen atoms. In a still further embodiment, one of R¹, R², and R³ is hydrogen; or any two adjacent ones of R¹, R², and R³, together with the carbon atoms to which they are attached, form a 5-membered heterocyclic ring containing two oxygen atoms.

In a further embodiment, R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms. In a yet further embodiment, one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms. In a still further embodiment, one of R⁴, R⁵, and R⁶ is hydrogen; or any two adjacent ones of R⁴, R⁵, and R⁶, together with the carbon atoms to which they are attached, form a 5-membered heterocyclic ring containing two oxygen atoms.

In a further embodiment, the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate. Preferably, the pharmaceutically acceptable salt of the compound of formula (I) is selected from a maleate and a hydrochloride. More preferably, the pharmaceutically acceptable salt of the compound of formula (I) is a hydrochloride.

In a further embodiment, the compound of formula (I) is selected from: 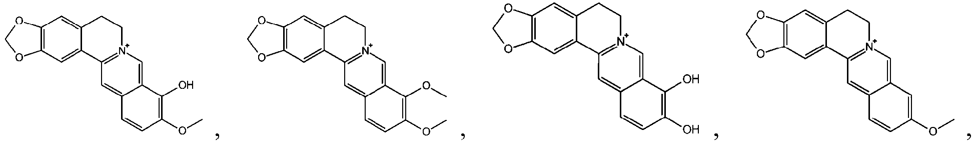 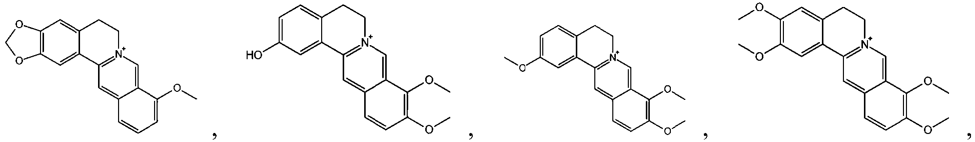 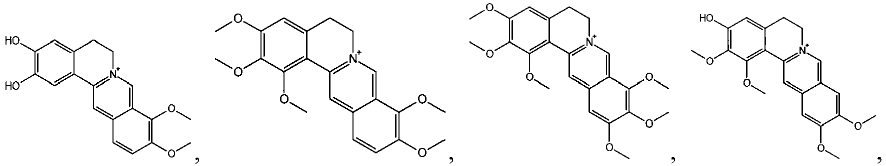 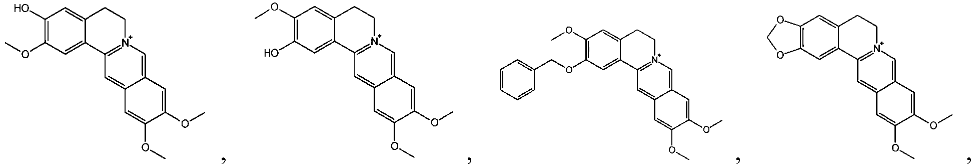 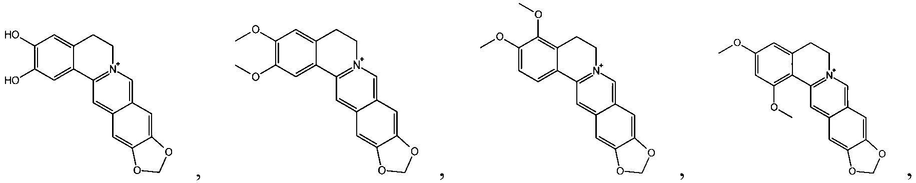 and

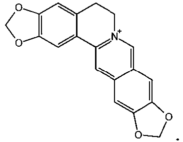

In a further embodiment, the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, tauroursodeoxycholic acid, etc.

In a further embodiment, the molar ratio of the component (a) to the component (b) is 1:1000-1000:1. In a further embodiment, the molar ratio of the component (a) to the component (b) is 1:100-100:1. Preferably, the molar ratio of the component (a) to the component (b) is 1:90-90:1, 1:80-80:1, 1:70-70:1, 1:60-60:1, 1:50-50:1, 1:40-40:1, 1:30-30:1, 1:20-20:1, or 1:10-10:1. More preferably, the molar ratio of the component (a) to the component (b) is 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or 10:1, or a numerical value between any two of these ratios. In some embodiments, the molar ratio of the component (a) to the component (b) is 1:5-5:1, preferably 1:2-2:1, and more preferably 1:1-2:1. In some embodiments, the molar ratio of the component (a) to the component (b) is 1:1 or 2:1.

In a further embodiment, the liver disease is selected from a cholestatic liver disease (chronic cholestatic liver disease), primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma. Preferably, the liver disease is primary sclerosing cholangitis (PSC) or primary biliary cirrhosis (PBC). More preferably, the primary sclerosing cholangitis (PSC) or primary biliary cirrhosis (PBC) is accompanied by inflammatory bowel disease as a complication.

In some embodiments, the inflammatory bowel disease is a complication arising from primary sclerosing cholangitis (PSC) and/or primary biliary cirrhosis (PBC).

In a further embodiment, the component (a) and the component (b) exhibit a synergistic effect in alleviating deoxycholic acid-induced hepatocyte damage.

In some aspects, the present disclosure provides the combination product, the pharmaceutical composition, the acid-base addition salt, or the compound of formula (I) or the pharmaceutically acceptable salt, the solvate, the hydrate, or the stereoisomer thereof according to any one of the preceding embodiments for use in treating, alleviating, and/or preventing a liver disease or inflammatory bowel disease.

In a further embodiment, the liver disease is selected from a cholestatic liver disease (chronic cholestatic liver disease), primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma. Preferably, the liver disease is primary sclerosing cholangitis (PSC) or primary biliary cirrhosis (PBC). More preferably, the primary sclerosing cholangitis (PSC) or primary biliary cirrhosis (PBC) is accompanied by inflammatory bowel disease as a complication.

In some embodiments, the inflammatory bowel disease is a complication arising from primary sclerosing cholangitis (PSC) and/or primary biliary cirrhosis (PBC).

### Mode of Administration

Administration of the compounds or salt forms in the combination of the present disclosure may be affected by any method capable of delivering the compound to the site of action. These methods include oral administration, intraduodenal administration, parenteral injection (including intravenous, subcutaneous, intramuscular, intravascular, or infusion), topical administration, and rectal administration.

The compounds or salt forms in the method or combination of the present disclosure may be formulated prior to administration. Preferably, the formulation will be suitable for the specific mode of administration. These compounds may be formulated with pharmaceutically acceptable carriers known in the art and administered in a variety of dosage forms known in the art. In the preparation of the pharmaceutical composition of the present disclosure, the active ingredient is typically mixed with a pharmaceutically acceptable carrier, or diluted with a carrier, or encapsulated within a carrier. Such carriers include, but are not limited to, solid diluents or fillers, excipients, sterile aqueous media, and various non-toxic organic solvents. Dosage unit forms or pharmaceutical compositions include tablets, capsules such as gelatin capsules, pills, powders, granules, aqueous and non-aqueous oral solutions and suspensions, lozenges, troches, hard candies, sprays, creams, salves, suppositories, pectins, gels, pastes, lotions, ointments, injectable solutions, elixirs, syrups, and parenteral solutions packaged in containers suitable for subdivision into individual doses.

Parenteral formulations include pharmaceutically acceptable aqueous or non-aqueous solutions, dispersions, suspensions, and emulsions, and sterile powders (for preparation thereof). Examples of carriers include water, ethanol, polyols (propylene glycol and polyethylene glycol), vegetable oils, and injectable organic esters such as ethyl oleate. Flowability may be maintained by using coatings such as lecithin or surfactants, or by maintaining an appropriate particle size. Exemplary parenteral administration forms include solutions or suspensions of the compounds of the present disclosure in sterile aqueous solutions such as aqueous propylene glycol solution or dextrose solution. Such dosage forms may be appropriately buffered, if necessary.

In addition, lubricants, such as magnesium stearate, sodium lauryl sulfate, and talc, are generally useful for tableting purposes. Similar types of solid compositions may also be used in soft and hard-filled gelatin capsules. Therefore, preferred materials include lactose (or milk sugar) and high molecular weight polyethylene glycols. When aqueous suspensions or elixirs are required for oral administration, the active compound therein may be combined with various sweetening or flavoring agents, coloring agents or dyes, and if necessary, emulsifying or suspending agents, as well as diluents such as water, ethanol, propylene glycol, glycerin, or combinations thereof.

Methods for preparing various pharmaceutical compositions using specific amounts of the active compound are known or apparent to those skilled in the art. For example, see Remington's Pharmaceutical Sciences, Mack Publishing Company, Easter, Pa., 15th Edition (1975).

### Kit

In some aspects, the present disclosure provides a kit for treating and/or preventing a liver disease or inflammatory bowel disease, which comprises the combination product, the acid-base addition salt, the pharmaceutical composition, or the compound of formula (I) or the pharmaceutically acceptable salt, the solvate, the hydrate, or the stereoisomer thereof according to any one of the preceding embodiments, and instructions for use in administering the therapeutic agent. In one embodiment, the instructions for use detail and define the mode of administration of the therapeutic agents, e.g., for simultaneous, concurrent, separate, or sequential administration of the therapeutic agents of the present disclosure. In one embodiment, the instructions for use detail and define the mode of administration of the therapeutic agents, for example, by specifying the days of administration for each therapeutic agent during a specific period of time.

In some aspects, the liver disease is selected from a cholestatic liver disease, primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma. Preferably, the liver disease is primary sclerosing cholangitis (PSC) or primary biliary cirrhosis (PBC). More preferably, the primary sclerosing cholangitis (PSC) or primary biliary cirrhosis (PBC) is accompanied by inflammatory bowel disease as a complication.

In some aspects, the inflammatory bowel disease is a complication arising from primary sclerosing cholangitis (PSC) and/or primary biliary cirrhosis (PBC).

### Examples

The present disclosure will be further described in detail with reference to the following examples, which should not be construed as limiting the present disclosure.

**Materials and reagents:** Unless otherwise stated, all reagents involved in the experimental section of the present disclosure are commercially available products. For example, berberine hydrochloride (compound B) and ursodeoxycholic acid (compound U) were purchased from Energy Chemical. Male C57BL/6 mice aged 10 weeks were purchased from Shanghai Lingchang Biotechnology Co., Ltd.

In obtaining the compounds and the corresponding analytical data described in the examples below, the following experimental and analytical protocols were followed, unless otherwise indicated.

LC-MS: Unless otherwise stated, the analytical LC-MS system used consisted of a Shimadzu LCMS-2020 with electrospray ionization (ESI) in positive ion detection mode, equipped with a 20ADXR pump, an SIL-20ACXR autosampler, a CTO-20AC column oven, an M20APDA detector, and an LCMS 2020MS detector. The column was HALO, specifically C18 30 mm × 5.0 mm, 2.7 µm. Mobile phase A was water containing 0.05% TFA, and mobile phase B was acetonitrile containing 0.05% TFA. The gradient was set as follows: changed from 5% mobile phase B to 100% (95%) in 2.0 min, maintained for 0.7 min, then returned to 5% mobile phase B in 0.05 min and maintained for 0.25 min. The column oven (CTO-20AC) was operated at 40.0 °C. The flow rate was 1.5 mL/min, and the injection volume was 1 µL. The PDA (SPD-M20A) detection range was from 190 nm to 400 nm. MS detector, configured with electrospray ionization as the ionizable source; acquisition mode: scanning; nebulizing gas flow rate: 1.5 L/min; drying gas flow rate: 15 L/min; detector voltage: tuned voltage ± 0.2 kV; DL temperature: 250°C; heating block temperature: 250 °C; scanning range: 90.00 m/z to 900.00 m/z. ELSD (Alltech 3300) detector parameters: drift tube temperature: 60 ± 5 °C; N2 flow rate: 1.8 ± 0.2 L/min. The mobile phase gradient was optimized for each compound. The calculated mass corresponds to the exact mass.

Preparative HPLC: Unless otherwise stated, preparative HPLC purification was performed using a Waters Auto purification system (2545-2767) equipped with a 2489 UV detector. The column was selected from one of the following: Waters C18, 19 mm × 150 mm, 5 µm; XBridge Prep OBD C18 column, 30 mm × 150 mm, 5 µm; XSelect CSH Prep C18 OBD column, 5 µm, 19 mm × 150 mm; XBridge Shield RP18 OBD column, 30 mm × 150 mm, 5 µm; XSelect CSH Fluoro-Phenyl, 30 mm × 150 mm, 5 µm; or YMC-Actus Triart C18, 30 mm × 150 mm, 5 µm. The mobile phase consisted of a mixture of acetonitrile (5%-95%) in an aqueous solution containing 0.1% FA or 10 mmol/L NH₄HCO₃. The flow rate was maintained at 25 mL/min, the injection volume was 1200 µL, and the UV detector used two channels: 254 nm and 220 nm. The mobile phase gradient was optimized for each compound.

Chiral chromatography: Chiral analytical chromatography was performed on one of the following: Chiralpak AS, AD; Chiralcel OD, OJ; Chiralpak IA, IB, IC, ID, IE, IF, IG, IH columns (Daicel Chemical Industries, Ltd.); (R,R)-Whelk-O1, (S,S)-Whelk-O1 columns (Regis technologies, Inc.); CHIRAL Cellulose-SB, SC, SA columns (YMC Co., Ltd.) as described; with different column dimensions (50 mm × 4.6 mm, 100 mm × 4.6 mm, 150 mm × 4.6 mm, 250 mm × 4.6 mm, 50 mm × 3.0 mm, 100 mm × 3.0 mm); using the percentage of ethanol in hexane (%Et/Hex) or isopropanol in hexane (%IPA/Hex) as an isocratic solvent system, or under supercritical fluid chromatography (SFC) conditions.

Normal phase flash chromatography: Unless otherwise stated, normal phase flash column chromatography (FCC) was performed on silica gel using a pre-packed silica gel column, with ethyl acetate (EtOAc)/hexane, ethyl acetate (EtOAc)/petroleum ether (b.p. 60-90 °C), CH₂Cl₂/MeOH, or CH₂Cl₂/10% 2 N NH₃ in MeOH as eluents.

¹H NMR: Unless otherwise stated, ¹H NMR spectra were obtained using a 400 MHz spectrometer (or a 300 MHz spectrometer) in a DMSO-d₆ solution. Nuclear magnetic resonance (NMR) spectroscopic characteristics refer to chemical shifts (δ) expressed in parts per million (ppm). Tetramethylsilane (TMS) was used as an internal standard in a DMSO-d₆ solution, and the residual CH₃OH peak or TMS was used as an internal standard in a CD₃OD solution. Coupling constants (J) are reported in Hertz (Hz). The nature of the shifts with respect to multiplicity is reported as s (singlet), d (doublet), t (triplet), q (quartet), dd (doublet of doublets), dt (doublet of triplets), m (multiplet), and br (broad).

Abbreviations used in this specification, particularly in the examples, are listed in the table below:

| | | |
|---|---|---|
| THF | = | Tetrahydrofuran |
| LAH | = | Lithium aluminum hydride |
| Compound B | = | Berberine hydrochloride 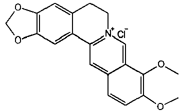 |
| Compound Y | = | 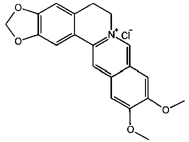 |
| Compound U | = | Ursodeoxycholic acid 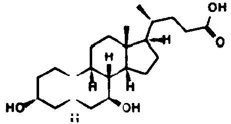 |
| Compound N | = | 24-Norursodeoxycholic acid 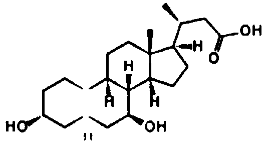 |
| DCM | = | Dichloromethane |
| DCE | = | Dichloroethane |
| MTBE | = | Methyl tert-butyl ether |
| TFA | = | Trifluoroacetic acid |
| YN salt | = | 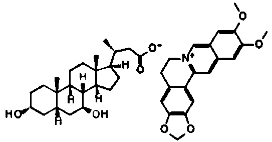 |

### Example 1. Synthesis of Compound Y

### Step 1. Synthesis of 2-(benzo[d][1,3]dioxol-5-yl)ethan-1-ol

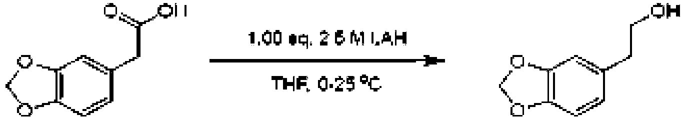

LAH (2.5 M, 1.00 L) was added dropwise to a solution of 1,3-benzodioxole-5-acetic acid (500 g, 2.78 mol) in THF (200 mL) at 0 °C, and then the mixture was stirred at 25 °C for 12 h under a N₂ atmosphere. The mixture was cooled to -10 to 0 °C, and then H₂O (95.0 mL), NaOH (15%, 95.0 mL), and H₂O (285 mL) were added sequentially. After 0.5 h, Na₂SO₄ (500 g) was added to the mixture, and the resulting mixture was stirred at 25 °C for 0.5 h. The mixture was filtered, and the filter cake was washed with THF (5.00 L). The filtrate was collected and concentrated to give the title compound (460 g, 2.77 mol, 99.7% yield) as a yellow oil.

### Step 2. Synthesis of 2-(benzo[d][1,3]dioxol-5-yl)ethane-1-pivalate

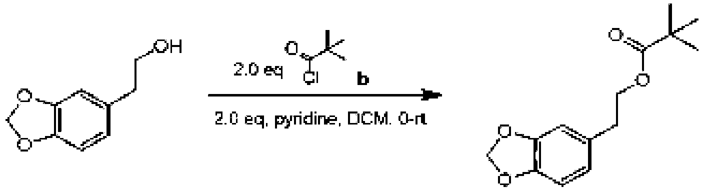

2-(Benzo[d][1,3]dioxol-5-yl)ethan-1-ol (455 g) and pyridine (435 g) were dissolved in DCM (2.00 L), and compound b (664 g) was added dropwise at 0 °C under a N₂ atmosphere. The mixture was stirred for 8 h. The residue was poured into water, and the aqueous phase was extracted with DCM. The organic phases were combined, washed with brine, dried, and concentrated. The residue was purified by silica gel chromatography to give the title compound (670 g) as a yellow oil.

HNMR (CDCl₃): δ6.78-6.70 (m, 2H), 6.68-6.64 (m, 1H), 6.10-5.58 (m, 2H), 4.22 (t, J = 6.94 Hz, 2H), 2.84 (t, J = 6.94 Hz, 2H), 1.20-1.16 (m, 9H).

### Step 3. Synthesis of 2-(6-acetyl-benzo[d][1,3]dioxol-5-yl)ethane-1-pivalate

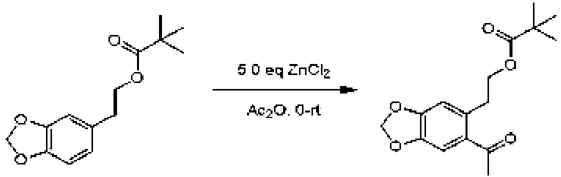

ZnCl₂ (1.01 kg) was added in one portion to a solution of 2-(benzo[d][1,3]dioxol-5-yl)ethane-1-pivalate (620 g) in Ac₂O (3.00 L) at 0 °C under N₂, and then the mixture was stirred at room temperature for 8 h. The reaction mixture was poured into water and then extracted with ethyl acetate. The organic phases were combined, washed with brine, dried, and concentrated. Purification was performed by silica gel chromatography to give the title compound (467 g) as a yellow oil.

HNMR (CDCl₃): *δ*7.23 (s, 1H), 6.75 (s, 1H), 6.78-6.70 (m, 1H), 6.02 (s, 2H), 4.26 (t, J = 6.63 Hz, 1H), 4.35-4.21(m, 1H), 3.17 (t, J = 6.63 Hz, 2H), 2.54 (s, 3H), 1.16 (s, 9H).

### Step 4. Synthesis of 2-(6-(2-(2-(1,3-dioxolan-2-yl)-4,5-dimethoxyphenyl)acetyl)benzo[d][1,3]dioxol-5-yl)ethyl pivalate

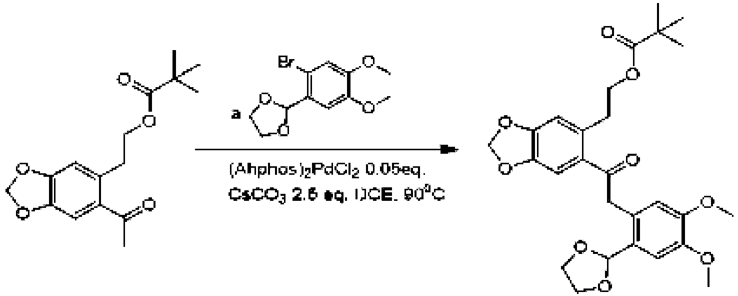

Cs₂CO₃ (451 g) and (Ahphos)₂PdCl₂ (58.8 g) were added in one portion to a solution of 2-(6-acetyl-benzo[d][1,3]dioxol-5-yl)ethane-1-pivalate (284 g) and compound a (200 g) in DCE at room temperature under a N₂ atmosphere. The mixture was left to react for 10 h with the temperature maintained at 90 °C. After the reaction was completed, the reaction system was slowly added dropwise to water and extracted with ethyl acetate. The organic phase was washed with brine, dried, and concentrated. Purification was performed by silica gel chromatography to give the title compound (310 g) as a yellow solid.

### Step 5. Synthesis of compound Y

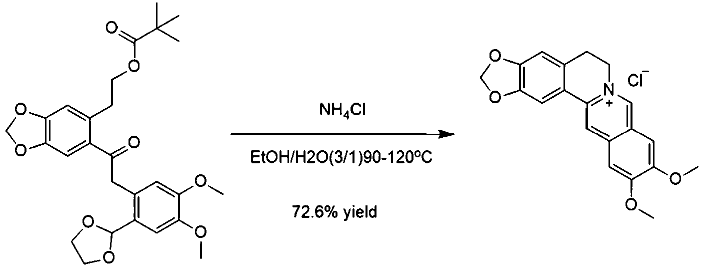

An NH₄Cl solution (3 M, 3.38 eq) was added in one portion to a solution of 2-(6-(2-(2-(1,3-dioxolan-2-yl)-4,5-dimethoxyphenyl)acetyl)benzo[d][1,3]dioxol-5-yl)ethyl pivalate (310 g) in EtOH at room temperature under a N₂ atmosphere, and then the mixture was left to react under high pressure at 120 °C for 24 h. After the reaction was completed, the mixture was cooled to 25 °C and filtered, and the filter cake was washed with MTBE. Purification was performed by silica gel chromatography to give the title compound (190 g).

HNMR (DMSO-*d₆*): *δ*9.73-9.41 (s, 1H), 8.91-8.58 (s, 1H), 7.79-7.66 (s, 2H), 7.62-7.55 (s, 1H), 7.15-7.04 (s, 1H), 6.23-6.09 (s, 2H), 4.86-4.69 (m, 2H), 4.10-4.04 (m, 3H), 4.02-3.97 (s, 3H), 3.25-3.15 (m, 2H).

### Example 2. Synthesis of Compound N

### Step 1. Synthesis of 3α,7β-diformyloxy-5β-cholan-24-oic acid

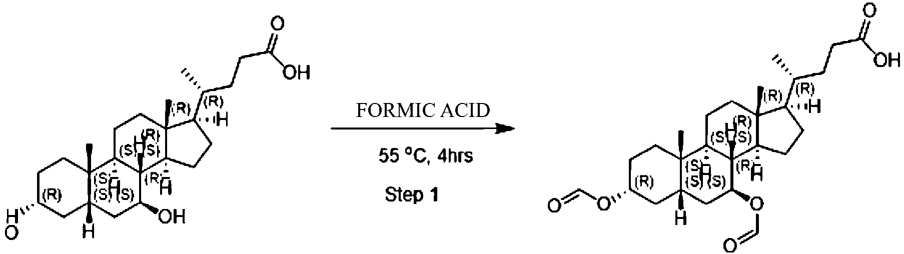

Deoxycholic acid (500 g) was dissolved in a formic acid solution (2.50 L) at room temperature, and then the mixture was stirred at 55 °C for 4 h under a N₂ atmosphere. After the reaction was completed, the reaction mixture was concentrated under vacuum to give the title compound as a crude product, which was directly used in the next step.

### Step 2. Synthesis of 3α,7β-diformyloxy-5β-23-cyano-cholestane

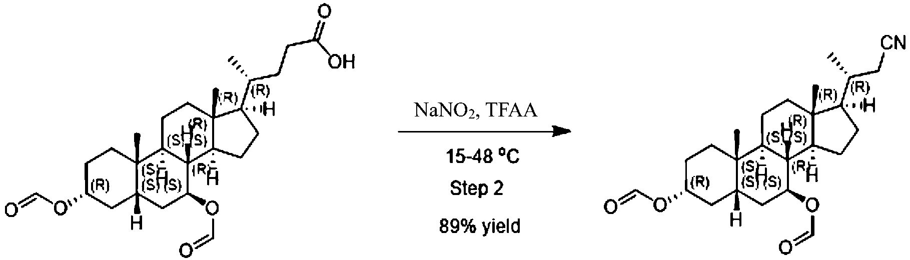

3α,7β-diformyloxy-5β-cholan-24-oic acid (660 g) and NaNO₂ (112 g) were added to a solution of TFAA (trifluoroacetic anhydride) (927 g) in TFA (2.00 L) at 0 °C, and the mixture was stirred for 1 h, then heated to 40 °C, and continuously stirred for 8 h. The mixture was then cooled to 25 °C, poured into water (6.00 L), stirred for 30 min, and extracted with ethyl acetate. The organic phases were combined, washed with brine, dried, and concentrated. Purification was performed by silica gel chromatography to give the title compound (550 g) as a yellow solid, which was directly used in the next step as a crude product.

### Step 3. Synthesis of compound N

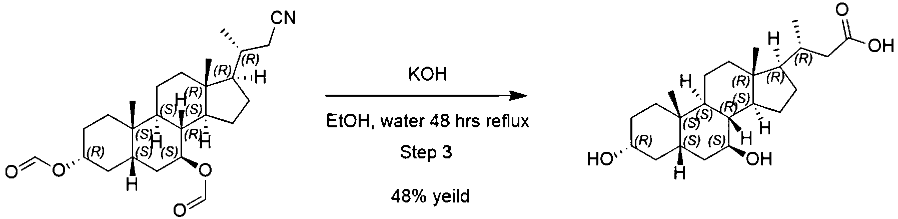

A 40% aqueous KOH solution (2.80 L) was added to a solution of 3α,7β-diformyloxy-5β-23-cyano-cholestane (300 g) dissolved in EtOH (2.80 L), and then the mixture was stirred in an autoclave at 110 °C for 8 h. After the reaction was completed, the mixture was cooled to 25 °C and poured into water (4.00 L). The aqueous phase was extracted with ethyl acetate. The resulting aqueous phase was then adjusted to pH = 2 and extracted with ethyl acetate, dried, and concentrated. Purification was performed by silica gel chromatography to give the title compound (130 g) as a yellow solid.

HNMR (MeOD): *δ*3.56-3.42 (m, 2H), 2.44 (d, J =11.52 Hz, 1H), 2.08 - 1.78 (m, 7H), 1.68 - 1.4 (m, 9H), 1.40 - 1.06(m, 7H), 1.02 (d, J =6.14 Hz, 3H), 0.97 (s, 3H), 0.78 - 0.72 (m, 3H).

### Example 3. Preparation and Characterization of YN Salt

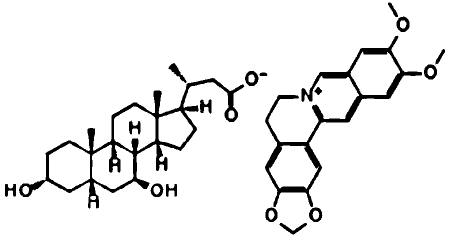

**Preparation of YN salt:** Supersaturated solutions of compound Y and compound N in water were separately prepared. The saturated solution of Y was added dropwise to the supersaturated solution of N in water, and the mixture was continuously stirred for 24 h, yielding a significant amount of solid. The resulting solid was filtered and dried to give the solid salt form.

**Characterization of YN salt:** The YN salt form solid exhibited a melting point of 224-226 °C. The ion ratio of Y to N in the YN salt form was 1:1.

HNMR (DMSO-*d₆*):*δ*11.96(s, 1H), 9.59(s,1H), 9.78(s, 1H), 7.73(s, 2H), 7.59(s, 1H), 7.10(s, 1H), 6.18(s, 2H), 4.80~4.77(t, 2H), 4.46~4.45(d, 1H), 4.07(s, 3H), 4.00(s, 3H), 3.89~3.88(d, 2H), 3.32~3.25(m, 2H), 3.21~3.18(t, 2H), 2.32~2.28(dd, 1H), 1.94~1.91(d, 1H), 1.87~1.81(m, 2H), 1.77~1.67(m, 3H), 1.65~1.62(m, 1H), 1.50~1.43(m, 3H), 1.42~1.26(m, 6H), 1.23~1.01(m, 6H), 0.95~0.91(dd, 4H), 0.87(s, 3H), 0.64(s, 3H).

### Example 4. Effects of Compound Y Monotherapy, Compound B Monotherapy, and Compound N Monotherapy on Protecting Hepatocytes from Deoxycholic Acid-Induced Hepatocyte Damage

First, 2.5 × 10⁴ HepG2 cells were seeded in a 96-well flat-bottom plate with an MEM medium containing 10% fetal bovine serum. The cells were cultured overnight in an incubator at 37 °C with 5% CO₂ for adhesion. Subsequently, various compounds at corresponding concentrations (compound Y: 100 µM; compound B: 100 µM; compound N: 50 µM) were incubated with the HepG2 cells in the incubator at 37 °C with 5% CO₂ for 24 h. All groups, except for the blank control group, were treated with 1 mM deoxycholic acid (DCA, a secondary bile acid that can directly induce hepatocyte damage) for 4 h. All groups were supplemented with 10 µL of CCK8 reagent and incubated under the incubator conditions for 1 h. The absorbance was measured at a wavelength of 450 nm using a microplate reader, which indirectly reflected the number of viable cells. All data were calculated and presented as percentages relative to the absorbance measured in the blank control group not treated with deoxycholic acid.

As shown in FIG. 1, compared to group 2 (column 2, deoxycholic acid-treated group), both compound B (column 3) and compound Y (column 4) resulted in a highly significant increase in cell viability; the compound N group (column 5) showed a trend toward improved cell viability, but the trend was not significant; and the effect of compound Y (column 4) in improving cell viability was significantly superior to that of compound B (column 3).

The above results show that, against deoxycholic acid-induced hepatocyte damage, the hepatocyte protective effect of the compound Y monotherapy was significantly superior to that of the compound B monotherapy and the compound N monotherapy.

### Example 5. Effects of Compound Y Monotherapy, Compound N Monotherapy, Compound Combination Y + N (1:1), and YN Salt on Protecting Hepatocytes from Deoxycholic Acid-Induced Hepatocyte Damage

First, 2.5 × 10⁴ HepG2 cells were seeded in a 96-well flat-bottom plate with an MEM medium containing 10% fetal bovine serum. The cells were cultured overnight in an incubator at 37 °C with 5% CO₂ for adhesion. Subsequently, various compounds at corresponding concentrations (compound Y: 50 µM; compound N: 50 µM; compound combination Y + N: 50 µM + 50 µM (molar ratio: 1:1); YN salt: 50 µM) were incubated with the HepG2 cells in the incubator at 37 °C with 5% CO₂ for 24 h. All groups, except for the blank control group, were treated with 1 mM deoxycholic acid (DCA, a secondary bile acid that can directly induce hepatocyte damage) for 4 h. All groups were supplemented with 10 µL of CCK8 reagent and incubated under the incubator conditions for 1 h. Then, the absorbance was measured at a wavelength of 450 nm using a microplate reader, which indirectly reflected the number of viable cells. All data were calculated and presented as percentages relative to the absorbance measured in the blank control group not treated with deoxycholic acid.

As shown in FIG. 2, compared to group 2 (column 2, deoxycholic acid-treated group), the cell viability in the compound Y group (column 3) was significantly increased to 72%; the compound N group (column 4) showed a trend toward improved cell viability, but the trend was not significant; the cell viability in the YN salt group (column 6) and the compound combination Y +N group (column 5) was significantly increased to about 80%. The results show that compound Y, the compound combination Y + N, and the YN salt had significant protective effects against DCA-induced hepatocyte damage. The effect of the compound combination Y + N (column 5) on increasing cell viability was substantially comparable to that of the YN salt (column 6). Compared to group 3 (compound Y monotherapy, column 3) and group 4 (compound N monotherapy, column 4), the YN salt (column 6) was more effective in reducing deoxycholic acid-induced hepatocyte damage.

The above results show that, against deoxycholic acid-induced hepatocyte damage, the YN salt and the compound combination Y + N had significantly superior hepatocyte protective effects compared to the compound Y monotherapy and the compound N monotherapy.

### Example 6. Effects of Compound Y Monotherapy, Compound N Monotherapy, and Compound Combination Y + N (2:1) on Protecting Hepatocytes from Deoxycholic Acid-Induced Hepatocyte Damage

First, 2.5 × 10⁴ HepG2 cells were seeded in a 96-well flat-bottom plate with an MEM medium containing 10% fetal bovine serum. The cells were cultured overnight in an incubator at 37 °C with 5% CO₂ for adhesion. Subsequently, various compounds at corresponding concentrations (compound Y: 100 µM; compound N: 50 µM; compound combination Y + N: 100 µM + 50 µM (molar ratio: 2:1)) were incubated with the HepG2 cells in the incubator at 37 °C with 5% CO₂ for 24 h. All groups, except for the blank control group, were treated with 1 mM deoxycholic acid (DCA, a secondary bile acid that can directly induce hepatocyte damage) for 4 h. All groups were supplemented with 10 µL of CCK8 reagent and incubated under the incubator conditions for 1 h. The absorbance was measured at a wavelength of 450 nm using a microplate reader, which indirectly reflected the number of viable cells. All data were calculated and presented as percentages relative to the absorbance measured in the blank control group not treated with deoxycholic acid.

As shown in FIG. 3, compared to group 2 (column 2, deoxycholic acid-treated group), the cell viability in the compound Y group (column 3) and the compound combination Y + N group (column 5) was increased to above 80%, with a highly significant difference, indicating that they had significant protective effects against DCA-induced hepatocyte damage.

### Example 7. Effects of Compound Combination Y + N (2:1) and Compound Combination B + N (2:1) on Protecting Hepatocytes from Deoxycholic Acid-Induced Hepatocyte Damage

First, 2.5 × 10⁴ HepG2 cells were seeded in a 96-well flat-bottom plate with an MEM medium containing 10% fetal bovine serum. The cells were cultured overnight in an incubator at 37 °C with 5% CO₂ for adhesion. Subsequently, various compounds at corresponding concentrations (compound combination Y + N: 100 µM + 50 µM (molar ratio: 2:1); compound combination B + N: 100 µM + 50 µM (molar ratio: 2:1)) were incubated with the HepG2 cells in the incubator at 37 °C with 5% CO₂ for 24 h. All groups, except for the blank control group, were treated with 1 mM deoxycholic acid (DCA, a secondary bile acid that can directly induce hepatocyte damage) for 4 h. All groups were supplemented with 10 µL of CCK8 reagent and incubated under the incubator conditions for 1 h. The absorbance was measured at a wavelength of 450 nm using a microplate reader, which indirectly reflected the number of viable cells. All data were calculated and presented as percentages relative to the absorbance measured in the blank control group not treated with deoxycholic acid.

As shown in FIG. 4, compared to group 2 (column 2, deoxycholic acid-treated group), group 3 (compound combination B + N) showed a trend toward improved cell viability, but the trend was not significant; group 4 (compound combination Y + N) exhibited a highly significant increase in cell viability; and the effect of the compound combination of Y + N in increasing cell viability was significantly superior to that of the compound combination of B + N.

The above results show that, against deoxycholic acid-induced hepatocyte damage, the compound combination Y + N had an excellent hepatocyte protective effect and was significantly superior to the compound combination B + N.

### Example 8. Inhibitory Effects of Compound Y Monotherapy, Compound N Monotherapy, and Compound Combination Y + N on Proliferation of CD4-Positive and CD8-Positive T Cells

It has been reported that overly active proliferation of CD4⁺ T cells and CD8⁺ T cells is involved in the pathogenesis of liver diseases such as primary sclerosing cholangitis and primary biliary cirrhosis (Tanja Schoknecht et al., CD4+ T cells from patients with primary sclerosing cholangitis exhibit reduced apoptosis and down-regulation of proapoptotic Bim in peripheral blood, J Leukoc Biol. 2017 Feb; 101(2): 589-597. doi: 10.1189/jlb.5A1015-469R. Epub 2016 Sep 14.; Aliya F Gulamhusein et al., Primary biliary cholangitis: pathogenesis and therapeutic opportunities, Nat Rev Gastroenterol Hepatol. 2020 Feb; 17(2): 93-110.). To observe the inhibitory effects of the compounds, the compound combination, or the salt of the present disclosure on immune cell infiltration or proliferation, their effects in inhibiting the proliferation of CD4-positive and CD8-positive T cells were further analyzed.

First, a certain amount of human peripheral blood mononuclear cells (PBMCs) were incubated with 5 µM CellTrace cell proliferation assay reagent in an incubator at 37 °C with 5% CO₂ in the dark for 20 min. Then, 1.5 × 10⁵ CellTrace-stained PBMCs were seeded in a 96-well U-bottom plate and treated with Anti-CD3/CD28 antibody-coupled magnetic beads at a ratio of 1:1 (cell count:magnetic bead count) to induce differentiation into T cells. Subsequently, various compounds at corresponding concentrations (compound Y: 200 µM; compound N: 100 µM; compound combination Y + N: 200 µM + 100 µM; control group: UDCA (100 µM)) were co-incubated with the above-treated cells in the incubator at 37 °C with 5% CO₂ for 72 h, followed by analysis by a flow cytometer. Specifically, the test cells were first treated with a Live/dead dye (633 nm, 1:1000 dilution) at 4 °C for 30 min to distinguish dead cells from live cells. Subsequently, the above test cells were simultaneously co-incubated with wavelength dyes anti-CD45-PerCPCy5.5, anti-CD4-AF700, and anti-CD8-FITC at room temperature for 30 min to distinguish different series of T cells (CD4-positive T cells and CD8-positive T cells). After appropriate washing, the above-stained cells were analyzed by a flow cytometer at different wavelengths. Subsequent cell analysis refers to the analysis of live cells that tested positive after staining with the Live/dead dye. CD4-positive T cells refer to cells positive for staining with anti-CD45-PerCPCy5.5 and anti-CD4-AF700 dyes; CD8-positive T cells refer to cells positive for staining with anti-CD45-PerCPCy5.5 and anti-CD8-FITC dyes. Proliferation of CD4-positive and CD8-positive T cells was determined by the fluorescence of CellTrace dye.

As shown in FIG. 5, compared to the control group (DMSO, waveform diagram 1; from top to bottom: waveform diagrams 1-5), the peaks of CD4-positive T cell proliferation in the groups treated with the corresponding doses of compound Y (waveform diagram 2) and the compound combination Y + N (waveform diagram 4) exhibited an overall rightward shift, indicating that the proliferation of CD4-positive T cells in these groups was significantly inhibited. Similarly, as shown in FIG. 6, compared to the control group (DMSO, waveform diagram 1), the peaks of CD8-positive T cell proliferation in the groups treated with compound Y (waveform diagram 2) and the compound combination Y + N (waveform diagram 4) exhibited an overall rightward shift, indicating that the proliferation of CD8-positive T cells in these groups was significantly inhibited.

The above results show that, for the CD4-positive and CD8-positive T cells derived from Anti-CD3/CD28-induced differentiation of human PBMCs, compound Y and the compound combination Y + N had significant inhibitory effects on the proliferation of CD4-positive and CD8-positive T cells, and their effects were significantly superior to those of compound N and compound U (UDCA), indicating that compound Y and the compound combination Y + N possess potential inhibitory effects on liver and bile duct inflammation.

### Example 9. Effects of Compound Y and Compound B on Serological Indicators in Mice Subjected to Intragastric Administration of α-Naphthylisothiocyanate (ANIT)

In order to verify the technical effects observed in the *in vitro* cell models, this experiment further examined the effects of compound Y and compound B on serological indicators in a mouse model subjected to intragastric administration of α-naphthylisothiocyanate (ANIT). Fifty-four female C57BL/6 mice aged 6-8 weeks were purchased from Shanghai Lingchang Biotechnology Co., Ltd. After one week of acclimatization, the mice were divided into 6 groups according to body weight, and this time point was designated as day -3. The test compound in each group was intragastrically administered starting from day -3. After three consecutive days of administration, modeling was performed by intragastric administration of ANIT at a dose of 50 mg/kg, and this time point was designated as day 0. During modeling, the healthy group (control) received a single oral dose of pure olive oil. Throughout the entire experimental period, both the healthy group (control) and the model group (ANIT) were given corresponding doses of vehicle (sodium carboxymethylcellulose) according to body weight. The mice were observed daily for health status and body weight changes. The experimental endpoint was day 2; prior to euthanasia, the mice were fasted for 6 h, and cardiac blood collection and liver sample acquisition were performed under anesthesia. The primary indicators were serological changes including AST, ALT, and ALP. The specific grouping and the administration dose for each group are shown in the table below:

| Group | Test compound | Administration dose (mg/kg) | Mode of administration | Frequency of administration | Total duration |
|---|---|---|---|---|---|
| 1 | Healthy group (vehicle) | - | Intragastric | Twice daily | |
| 2 | Model group (vehicle) | - | Intragastric | Twice daily | Day -3 to day 2 |
| 3 | Y | 100 | Intragastric | Twice daily | |
| 4 | Y | 50 | Intragastric | Twice daily | |
| 5 | B | 100 | Intragastric | Twice daily | |
| 6 | B | 50 | Intragastric | Twice daily | |

As shown in FIG. 7, compared to the model group (ANIT, group 2), the high-dose compound Y group (100 mg/kg, group 3) exhibited significantly reduced serum alkaline phosphatase (ALP) levels, and the low-dose compound Y group (50 mg/kg, group 4) also exhibited reduced serum ALP levels, while compound B increased serum ALP levels in a dose-dependent manner, indicating that the effect of compound Y in reducing serum ALP levels was significantly superior to that of compound B. As shown in FIG. 8, compared to the model group (ANIT, group 2), the high-dose compound Y group (100 mg/kg, group 3) exhibited significantly reduced serum ALT levels, and the low-dose compound Y group (50 mg/kg, group 4) also exhibited reduced serum ALT levels, while compound B increased serum ALT levels in a dose-dependent manner, indicating that the effect of compound Y in reducing serum ALT levels was significantly superior to that of compound B. As shown in FIG. 9, compared to the model group (ANIT, group 2), compound Y reduced serum AST levels in a dose-dependent manner, while compound B increased serum AST levels in a dose-dependent manner, indicating that the effect of compound Y in reducing serum AST levels was significantly superior to that of compound B.

The above results show that compound Y significantly reduced serum ALP and serum ALT levels, decreased serum AST levels, and could mitigate ANIT-induced cholestasis and biliary epithelial cell damage and protect the liver from cholestasis-induced injury, and its effect was significantly superior to that of compound B.

### Example 10. Effects of Various Compound Combinations on Serological Indicators in Mice Subjected to Intragastric Administration of α-Naphthylisothiocyanate (ANIT)

In order to verify the technical effects observed in the DDC model, this experiment further examined the effects of various compound combinations on serological indicators in a mouse model subjected to intragastric administration of α-naphthylisothiocyanate (ANIT). Fifty-six female C57BL/6 mice aged 6-8 weeks were purchased from Shanghai Lingchang Biotechnology Co., Ltd. After one week of acclimatization, the mice were divided into 7 groups according to body weight, and this time point was designated as day -3. The compound in each group was intragastrically administered starting from day -3. After three consecutive days of administration, modeling was performed by intragastric administration of ANIT at a dose of 50 mg/kg, and this time point was designated as day 0. The normal control group received a single oral dose of pure olive oil. Throughout the entire experimental period, both the healthy group (control) and the model group (ANIT) were given corresponding doses of vehicle (sodium carboxymethylcellulose) according to body weight. The mice were observed daily for health status and body weight changes. The experimental endpoint was day 2; prior to euthanasia, the mice were fasted for 6 h, and cardiac blood collection and liver sample acquisition were performed under anesthesia. The primary indicators were serological changes including AST, ALT, and ALP. The specific grouping and the administration dose for each group are shown in the table below:

| Group | Test compound | Administration dose (mg/kg) | Mode of administration | Frequency of administration | Total duration |
|---|---|---|---|---|---|
| 1 | Healthy group (vehicle) | - | Intragastric | Twice daily | |
| 2 | Model group (vehicle) | - | Intragastric | Twice daily | Day -3 to day 2 |
| 3 | U | 100 | Intragastric | Twice daily | |
| 4 | B+U | 100+50 | Intragastric | Twice daily | |
| 5 | B+N | 100+50 | Intragastric | Twice daily | |
| 6 | Y+N | 100+50 | Intragastric | Twice daily | |
| 7 | Y+U | 100+50 | Intragastric | Twice daily | |

As shown in FIG. 10, compared to the model group (ANIT group, group 2), the positive control group (UDCA (U), group 3) exhibited reduced serum alkaline phosphatase (ALP) levels, while the compound combination Y + N (group 6) and the compound combination Y + U (group 7) both significantly reduced serum ALP levels by about 2 times, significantly alleviating primary sclerosing cholangitis; and the effect of the compound combination Y + N (group 6) was significantly superior to that of the compound combination B + N (group 5). As shown in FIG. 11, compared to the model group (ANIT group, group 2), the positive control UDCA (U, group 3) significantly increased serum ALT levels, while the compound combination Y + N (group 6) significantly reduced serum ALT levels, indicating that the compound combination Y + N had a significant hepatoprotective effect. As shown in FIG. 12, compared to the model group (ANIT group, group 2), the positive control UDCA (U, group 3) significantly increased serum AST levels, while the compound combination Y + N (group 6) significantly reduced serum AST levels, indicating that the compound combination Y + N had a significant hepatoprotective effect.

The above results show that the compound combination Y + N significantly reduced serum ALP levels and mitigated ANIT-induced cholestasis and biliary epithelial cell damage, and its effect was significantly superior to that of the compound combination B + N. In addition, the compound combination Y + N also significantly reduced serum ALT and AST levels and protected the liver from cholestasis-induced injury, and its effect was significantly superior to that of the positive control U.

### Example 11. Effects of Compound Y Monotherapy (Y), Compound N Monotherapy (N), Compound Combination Y + N, and Positive Control UDCA (U) on Serological Indicators in Mice Fed with Feed Containing 0.1% Diethyl 1,4-Dihydro-2,4,6-Trimethyl-3,5-Pyridinedicarboxylate (DDC Model)

Seventy-one male C57BL/6 mice aged 10 weeks were purchased from Shanghai Lingchang Biotechnology Co., Ltd. After one week of acclimatization of mice, the healthy control group was fed with normal feed, while the other groups were fed with feed containing 0.1% 3,5-diethoxycarbonyl-1,4-dihydrocollidine (DDC), and this time point was recorded as day 0. The compound in each group was intragastrically administered starting from day 0. Throughout the entire experimental period, both the healthy group (control) and the model group (DDC) were given corresponding doses of vehicle (sodium carboxymethylcellulose) according to body weight. The mice were observed daily for health status and body weight changes. The experimental endpoint was day 20. Prior to euthanasia, the mice were fasted for 6 h, and cardiac blood collection and liver sample acquisition were performed under anesthesia. The primary indicators were serological changes including AST, ALT, and ALP; pathological analysis was performed by HE staining. The specific grouping and the administration dose for each group are shown in the table below:

| Group | Test compound | Administration dose (mg/kg) | Mode of administration | Frequency of administration | Total duration |
|---|---|---|---|---|---|
| 1 | Healthy group (vehicle) | - | Intragastric | Twice daily | Day 0 to day 21 |
| 2 | Model group (vehicle) | - | Intragastric | Twice daily | |
| 3 | U | 100 | Intragastric | Twice daily | |
| 4 | Y | 100 | Intragastric | Twice daily | |
| 5 | N | 56.5 | Intragastric | Twice daily | |
| 6 | Y+N | 100+56.5 | Intragastric | Twice daily | |
| 7 | Y | 60 | Intragastric | Twice daily | |
| 8 | N | 34.5 | Intragastric | Twice daily | |
| 9 | Y+N | 60+34.5 | Intragastric | Twice daily | |

As shown in FIG. 13, compared to the model group (DDC group, group 2), all experimental groups, except for the low-dose compound Y (60 mg/kg) group, exhibited significantly reduced serum alkaline phosphatase (ALP) levels. Compared to the model group (DDC group, group 2) and the positive control group (U, group 3), the compound combination Y + N experimental groups (group 6 and group 9) showed a dose-dependent reduction in serum ALP levels. Compared to 100 mg/kg compound Y (group 4) and 56.5 mg/kg compound N (group 5), the high-dose compound combination Y + N (group 6) more significantly reduced serum ALP levels.

The above results show that, compared to compound Y or compound N alone, the compound combination Y + N significantly reduced serum ALP levels.

### Example 12. Effects of Various Compound Combinations on Serological Indicators in Mice Fed with Feed Containing 0.1% Diethyl 1,4-Dihydro-2,4,6-Trimethyl-3,5-Pyridinedicarboxylate (DDC Model)

Fifty-six male C57BL/6 mice aged 10 weeks were purchased from Shanghai Lingchang Biotechnology Co., Ltd. After one week of acclimatization of mice, the healthy control group was fed with normal feed, while the other groups were fed with feed containing 0.1% 3,5-diethoxycarbonyl-1,4-dihydrocollidine (DDC), and this time point was recorded as day 0. The compound in each group was intragastrically administered starting from day 0. Throughout the entire experimental period, both the healthy group (control) and the model group (DDC) were given corresponding doses of vehicle (sodium carboxymethylcellulose) according to body weight. The mice were observed daily for health status and body weight changes. The experimental endpoint was day 20. Prior to euthanasia, the mice were fasted for 6 h, and cardiac blood collection and liver sample acquisition were performed under anesthesia. The primary indicators were serological changes including AST, ALT, and ALP; pathological analysis was performed by HE staining. The specific grouping and the administration dose and dosing schedule for each group are shown in the table below:

| Group | Test compound | Administration dose (mg/kg) | Mode of administration | Frequency of administration | Total duration |
|---|---|---|---|---|---|
| 1 | Healthy group (vehicle) | - | Intragastric | Twice daily | |
| 2 | Model group (vehicle) | - | Intragastric | Twice daily | Day 0 to day 21 |
| 3 | U | 100 | Intragastric | Twice daily | |
| 4 | B+U | 100+100 | Intragastric | Twice daily | |
| 5 | B+N | 100+100 | Intragastric | Twice daily | |
| 6 | Y+N | 100+100 | Intragastric | Twice daily | |
| 7 | Y+U | 100+100 | Intragastric | Twice daily | |

As shown in FIG. 14, compared to the model group (DDC group, group 2), all treatment groups exhibited significantly reduced serum alkaline phosphatase (ALP) levels; among them, the compound combination B + N (group 5) and the compound combination Y + N (group 6) reduced serum ALP levels by more than 2 times compared to the model group (DDC group, group 2). Compared to the positive control group (UDCA (U), group 3), the compound combination B + N (group 5) and the compound combination Y + N (group 6) more significantly reduced serum ALP levels. As shown in FIGs. 15 and 16, compared to the model group (DDC group, group 2), the compound combination Y + N (group 6) could significantly reduce alanine transaminase (ALT) and aspartate transaminase (AST) levels in serum, indicating that it had a significant effect in mitigating liver injury. As shown in FIG. 17, compared to the model group (DDC group, group 2), the groups treated with the positive control U (group 3), the compound combination B + N (group 5), the compound combination Y + N (group 6), and the compound combination Y + U (group 7) all exhibited a significant reduction in the bile pigment-positive area of the liver, indicating that they significantly reduced bile pigment levels in the liver. As shown in FIG. 18, compared to the model group (DDC group, group 2), only the compound combination Y + N (group 6) significantly reduced collagen levels in the liver, indicating that it significantly inhibited hepatic fibrosis, and the effect of the compound combination Y + N (group 6) was significantly superior to that of the compound combination B + N (group 5).

The above results show that the compound combination Y + N had the effect of reducing serum ALP levels, and the effect was significantly superior to that of the positive control UDCA. The compound combination Y + N could also significantly reduce serum ALT and AST levels, indicating that the compound combination Y + N mitigated cholestasis-induced liver injury. In addition, from the perspective of histopathology, the compound combination Y + N could also significantly reduce the bile pigment deposition area and collagen area in the liver, and its effect was significantly superior to that of the compound combination B + N.

### Example 13. Effect of Compound Combination Y + N on Inflammatory Bowel Disease Induced in Mice Fed with Drinking Water Containing Dextran Sulfate Sodium (DSS)

Cholestatic liver diseases are mostly accompanied by inflammatory bowel disease; for example, over 50% of patients with primary sclerosing cholangitis also suffer from inflammatory bowel disease (Guidelines for the Diagnosis and Treatment of Primary Sclerosing Cholangitis (2021)), or 80% of patients suffer from inflammatory bowel disease (Mago, S. and G. Y. Wu (2020). "Primary Sclerosing Cholangitis and Primary Biliary Cirrhosis Overlap Syndrome: A Review." J Clin Transl Hepatol 8(3): 336-346.). This experiment was intended to verify the regulatory effect of the compound combination Y + N on inflammatory bowel disease. Thirty female C57BL/6 mice aged 6-8 weeks were purchased from Shanghai Lingchang Biotechnology Co., Ltd. After one week of acclimatization, the mice were divided into 3 groups according to body weight, and this time point was designated as day 0. Except for the mice in the control group, the mice in the model group and the compound combination Y + N group were provided with 3% DSS solution from day 0 to day 7. The compound combination Y + N group was given the drug from day 0 to day 7, while the control group and the model group were subjected to intragastric administration of the corresponding vehicle. Day 8 was designated as the experimental endpoint. During the experiment, the mice were observed daily for health status, body weight changes, stool appearance, presence of bloody stools, etc. Prior to euthanasia, the mice were fasted for 6 h, and colon sample acquisition was performed under anesthesia.

As shown in FIG. 19, compared to the control group, the model group (DSS) exhibited a significant decrease in body weight starting from day 4, which persisted until the experimental endpoint; compared to the model group (DSS), the mice in the compound combination Y + N exhibited a significant increase in body weight starting from day 4, indicating that the compound combination Y + N could significantly improve the survival status of mice with inflammatory bowel disease. The DAI score is a comprehensive assessment mode for evaluating inflammatory bowel disease by combining body weight, stool appearance, and the presence of bloody stools. As shown in FIG. 20, starting from day 3 of DSS drinking, the DAI score of the mice in the DSS group was significantly higher than that in the control group; as the DSS drinking continued, the DAI score of the mice in the DSS model group reached as high as 9; in contrast, the DAI score of the mice in the compound combination Y + N group was significantly lower than that in the DSS model group from day 4 onward, indicating that the compound combination Y + N could significantly ameliorate the overall symptoms of inflammatory bowel disease in mice. Inflammatory bowel disease may cause colon shortening. As shown in FIG. 21, the colon length of the mice in the model group (DSS) was reduced by about 30%, while the colon length of the mice in the compound combination Y + N group could be significantly maintained, indicating that the compound combination Y + N could significantly ameliorate the symptom of colon shortening associated with inflammatory bowel disease in mice. In conclusion, the compound combination Y + N significantly ameliorated DSS drinking-induced ulcerative colitis in mice.

It should be understood that various modifications or changes may be made by those skilled in the art after reading the aforementioned content of the present disclosure, and these equivalent forms also fall within the scope defined by the appended claims of the present application.

## Claims

1. A combination product, **characterized in that** the combination product comprises:
(a) a compound of formula (I) or a pharmaceutically acceptable salt, a solvate, a hydrate, or a stereoisomer thereof: 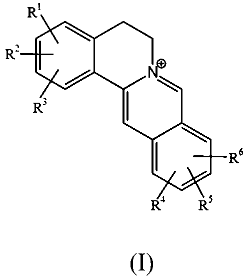
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N; and
(b) a bile acid or a derivative or an analog thereof, or a pharmaceutically acceptable salt, a solvate, a hydrate, or a stereoisomer thereof.

2. The combination product according to claim 1, **characterized in that** R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms.

3. The combination product according to claim 1 or 2, **characterized in that** R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms.

4. The combination product according to any one of the preceding claims, **characterized in that** one or two of R¹, R², and R³ are hydrogen; or any two adjacent ones of R¹, R², and R³, together with the carbon atoms to which they are attached, form a 5-membered heterocyclic ring containing one or more oxygen atoms.

5. The combination product according to any one of the preceding claims, **characterized in that** one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms.

6. The combination product according to any one of the preceding claims, **characterized in that** the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate, preferably a maleate or a hydrochloride, and more preferably a hydrochloride.

7. The combination product according to any one of the preceding claims, **characterized in that** the compound of formula (I) is selected from: 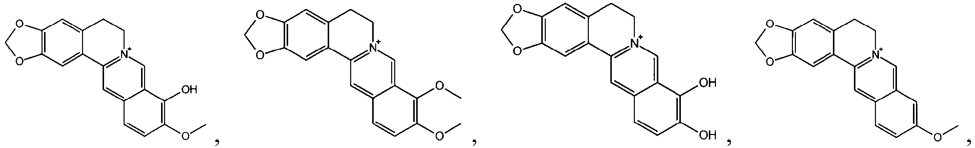 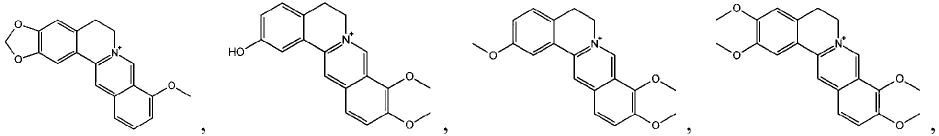 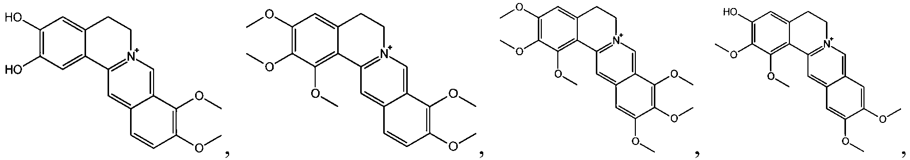 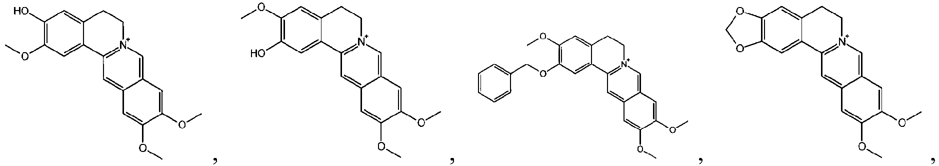 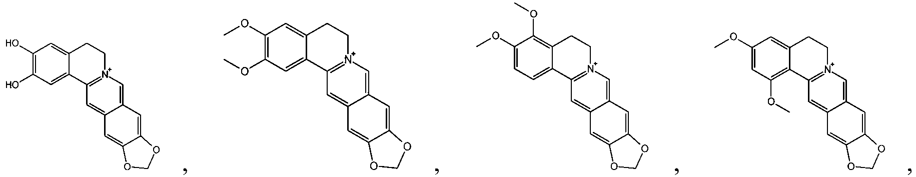 and

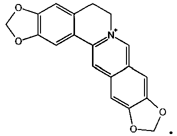

8. The combination product according to any one of the preceding claims, **characterized in that** the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, and tauroursodeoxycholic acid.

9. The combination product according to any one of the preceding claims, **characterized in that** the molar ratio of the component (a) to the component (b) is 1:1000-1000:1; preferably, the molar ratio of the component (a) to the component (b) is 1:5-5:1; more preferably, the molar ratio of the component (a) to the component (b) is 1:2-2:1; further preferably, the molar ratio of the component (a) to the component (b) is 1:1-2:1; even more preferably, the molar ratio of the component (a) to the component (b) is 1:1 or 2:1.

10. The combination product according to any one of the preceding claims, **characterized in that** the combination product is for use in treating and/or preventing a liver disease, preferably a chronic cholestatic liver disease.

11. The combination product according to any one of the preceding claims, **characterized in that** the liver disease is selected from a cholestatic liver disease, primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma.

12. The combination product according to any one of the preceding claims, **characterized in that** the component (a) and the component (b) exhibit a synergistic effect in alleviating deoxycholic acid-induced hepatocyte damage.

13. A pharmaceutical composition, **characterized in that** the pharmaceutical composition comprises:
(a) a compound of formula (I) or a pharmaceutically acceptable salt, a solvate, a hydrate, or a stereoisomer thereof: 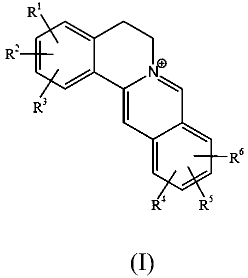
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N;
(b) a bile acid or a derivative or an analog thereof, or a pharmaceutically acceptable salt, a solvate, a hydrate, or a stereoisomer thereof; and
(c) a pharmaceutically acceptable carrier, excipient, and/or diluent.

14. The pharmaceutical composition according to claim 13, **characterized in that** R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms.

15. The pharmaceutical composition according to claim 13 or 14, **characterized in that** R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms.

16. The pharmaceutical composition according to any one of claims 13-15, **characterized in that** one or two of R¹, R², and R³ are hydrogen; or any two adjacent ones of R¹, R², and R³, together with the carbon atoms to which they are attached, form a 5-membered heterocyclic ring containing one or more oxygen atoms.

17. The pharmaceutical composition according to any one of claims 13-16, **characterized in that** one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms.

18. The pharmaceutical composition according to any one of claims 13-17, **characterized in that** the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate, preferably a maleate or a hydrochloride, and more preferably a hydrochloride.

19. The pharmaceutical composition according to any one of claims 13-18, **characterized in that** the compound of formula (I) is selected from: 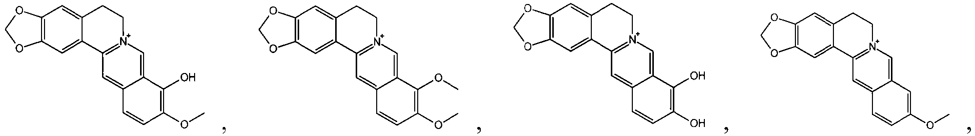 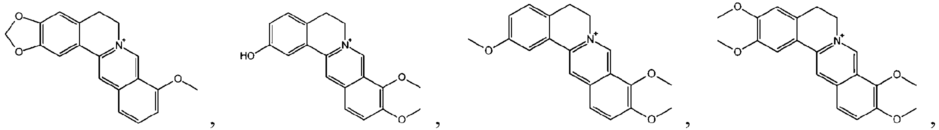 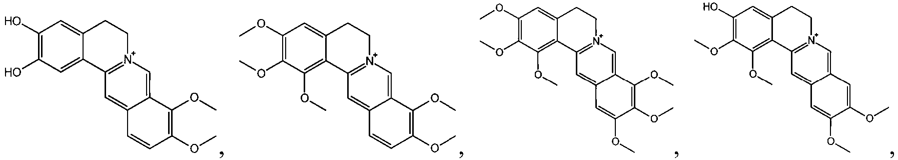 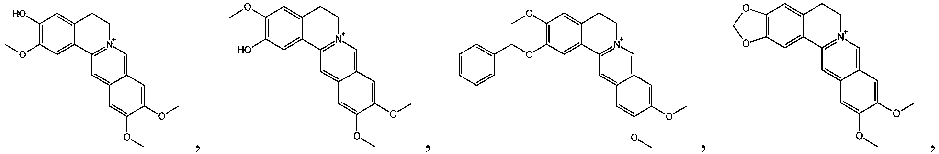 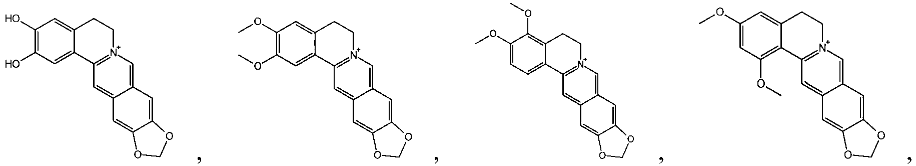 and

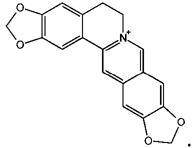

20. The pharmaceutical composition according to any one of claims 13-19, **characterized in that** the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, and tauroursodeoxycholic acid.

21. The pharmaceutical composition according to any one of claims 13-20, **characterized in that** the molar ratio of the component (a) to the component (b) is 1:1000-1000:1; preferably, the molar ratio of the component (a) to the component (b) is 1:5-5:1; more preferably, the molar ratio of the component (a) to the component (b) is 1:2-2: 1; further preferably, the molar ratio of the component (a) to the component (b) is 1:1-2:1; even more preferably, the molar ratio of the component (a) to the component (b) is 1:1 or 2:1.

22. The pharmaceutical composition according to any one of claims 13-21, **characterized in that** the pharmaceutical composition is for use in treating and/or preventing a liver disease or inflammatory bowel disease, preferably a chronic cholestatic liver disease.

23. The pharmaceutical composition according to any one of claims 13-22, **characterized in that** the liver disease is selected from a cholestatic liver disease, primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma.

24. The pharmaceutical composition according to any one of claims 13-23, **characterized in that** the component (a) and the component (b) exhibit a synergistic effect in alleviating deoxycholic acid-induced hepatocyte damage.

25. An acid-base addition salt of formula (II):
(A⁺)ₘ(B⁻)ₙ(C⁻)ₚ (II)
**characterized in that**
(a) A⁺ is a cationic moiety, which is a compound of formula (I): 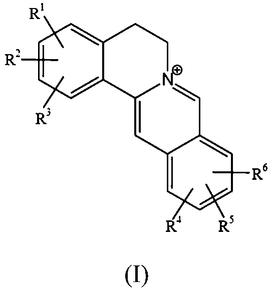
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N;
(b) B⁻ is an anionic moiety, which is a bile acid or a derivative or an analog thereof; and
(c) C⁻ is an acid anion;
wherein m, n, and p are each independently an integer selected from 1-6, such that the configuration of the salt reaches charge balance, and when m = n, p is 0.

26. The acid-base addition salt according to claim 25, **characterized in that** R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms.

27. The acid-base addition salt according to claim 25 or 26, **characterized in that** R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms.

28. The acid-base addition salt according to any one of claims 25-27, **characterized in that** one or two of R¹, R², and R³ are hydrogen; or any two adjacent ones of R¹, R², and R³, together with the carbon atoms to which they are attached, form a 5-membered heterocyclic ring containing one or more oxygen atoms.

29. The acid-base addition salt according to any one of claims 25-28, **characterized in that** one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms.

30. The acid-base addition salt according to any one of claims 25-29, **characterized in that** the compound of formula (I) is a cationic moiety selected from the group consisting of: 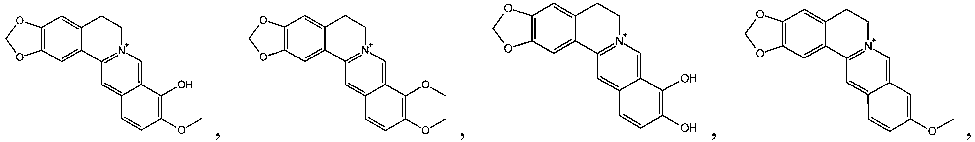 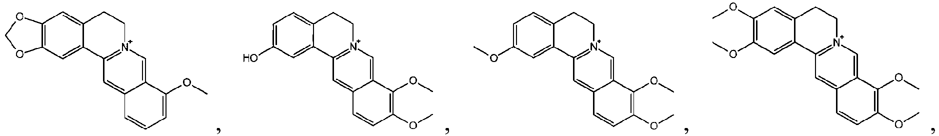 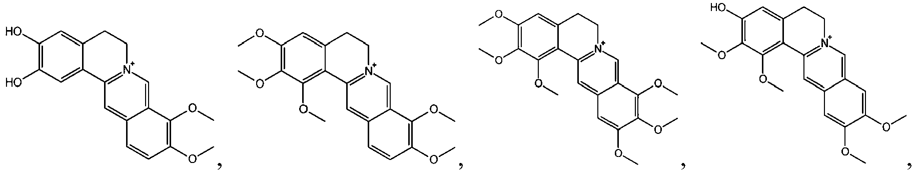 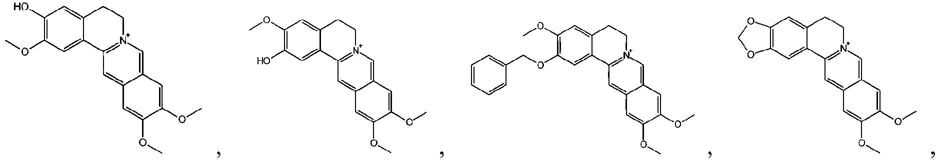 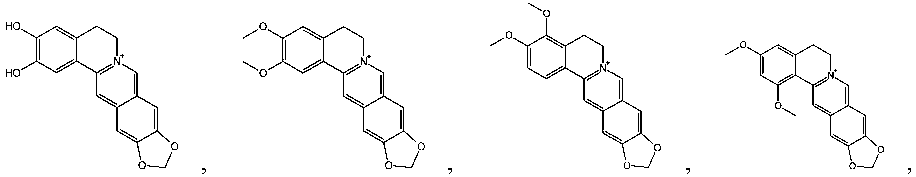 and

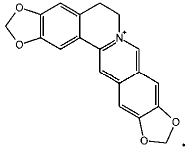

31. The acid-base addition salt according to any one of claims 25-30, **characterized in that** the component (b) is an anionic moiety selected from the group consisting of: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, and tauroursodeoxycholic acid.

32. The acid-base addition salt according to any one of claims 25-31, **characterized in that** m is 1, n is 1, and p is 0; or m is 2, n is 1, and p is 1.

33. The acid-base addition salt according to any one of claims 25-32, **characterized in that** the acid-base addition salt is for use in treating and/or preventing a liver disease or inflammatory bowel disease, preferably a chronic cholestatic liver disease.

34. The acid-base addition salt according to any one of claims 25 to 33, **characterized in that** the liver disease is selected from a cholestatic liver disease, primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma.

35. A pharmaceutical composition, **characterized in that** the pharmaceutical composition comprises the combination product according to any one of claims 1-12 or the acid-base addition salt according to any one of claims 25-34.

36. A method for treating and/or preventing a liver disease or inflammatory bowel disease, **characterized in that** the method comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding claims 1-35.

37. The method according to embodiment 36, **characterized in that** the liver disease is selected from a cholestatic liver disease, primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma.
37. A method for treating and/or preventing a liver disease or inflammatory bowel disease, **characterized in that** the method comprises:
regimen 1: administering to a patient in need thereof:
(a)a therapeutically and/or prophylactically effective amount of a compound of formula (I) or a pharmaceutically acceptable salt, a solvate, a hydrate, or a stereoisomer thereof: 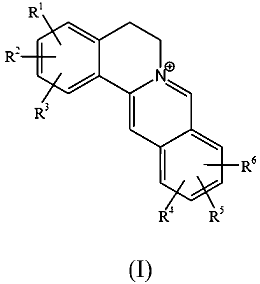
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N; or
regimen 2: administering to a patient in need thereof the following components simultaneously, concurrently, separately, or sequentially:
(a) a therapeutically and/or prophylactically effective amount of a compound of formula (I) or a pharmaceutically acceptable salt, a solvate, a hydrate, or a stereoisomer thereof: 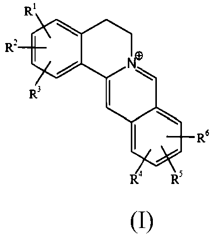
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N; and
(b) a therapeutically and/or prophylactically effective amount of a bile acid or a derivative or an analog thereof, or a pharmaceutically acceptable salt, a solvate, a hydrate, or a stereoisomer thereof.

38. The method according to claim 37, **characterized in that** R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms.

39. The method according to claim 37 or 38, **characterized in that** R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms.

40. The method according to any one of claims 37-39, **characterized in that** one or two of R¹, R², and R³ are hydrogen; or any two adjacent ones of R¹, R², and R³, together with the carbon atoms to which they are attached, form a 5-membered heterocyclic ring containing one or more oxygen atoms.

41. The method according to any one of claims 37-40, **characterized in that** one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms.

42. The method according to any one of claims 37-41, **characterized in that** the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate, preferably a maleate or a hydrochloride, and more preferably a hydrochloride.

43. The method according to any one of claims 37-42, **characterized in that** the compound of formula (I) is selected from:

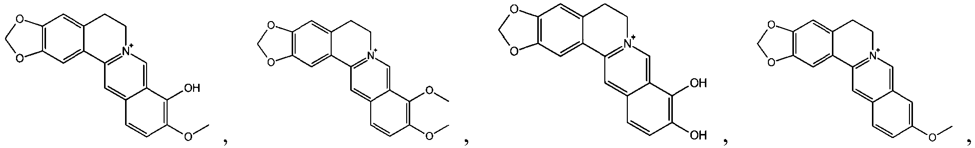

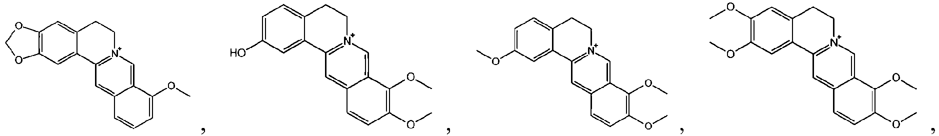

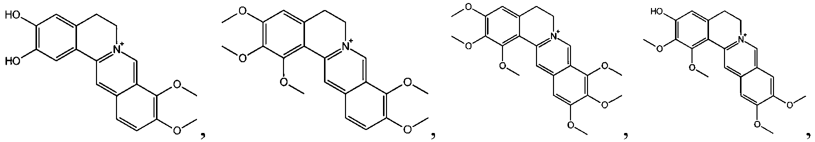

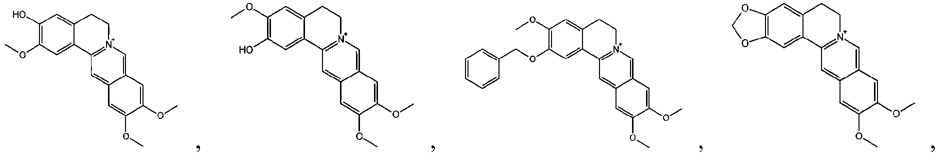

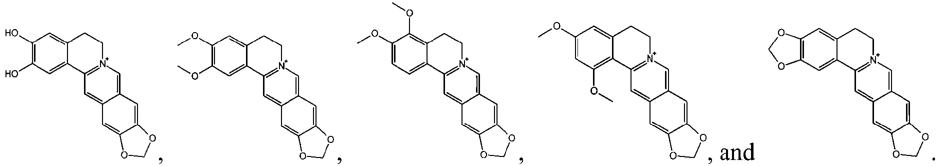

44. The method according to any one of claims 37-43, **characterized in that** the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, and tauroursodeoxycholic acid.

45. The method according to any one of claims 37-44, **characterized in that** the molar ratio of the component (a) to the component (b) is 1:1000-1000:1; preferably, the molar ratio of the component (a) to the component (b) is 1:5-5:1; more preferably, the molar ratio of the component (a) to the component (b) is 1:2-2:1; further preferably, the molar ratio of the component (a) to the component (b) is 1:1-2:1; even more preferably, the molar ratio of the component (a) to the component (b) is 1:1 or 2:1.

46. The method according to any one of claims 37-45, **characterized in that** the liver disease is selected from a cholestatic liver disease, primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma.

47. The combination product, the pharmaceutical composition, the acid-base addition salt, or the compound of formula (I) or the pharmaceutically acceptable salt, the solvate, the hydrate, or the stereoisomer thereof according to any one of claims 1-35, **characterized in that** the combination product, the pharmaceutical composition, the acid-base addition salt, or the compound of formula (I) or the pharmaceutically acceptable salt, the solvate, the hydrate, or the stereoisomer thereof is for use in treating, alleviating, and/or preventing a liver disease or inflammatory bowel disease.

48. The combination product, the pharmaceutical composition, the acid-base addition salt, or the compound of formula (I) or the pharmaceutically acceptable salt, the solvate, the hydrate, or the stereoisomer thereof according to claim 47, **characterized in that** the liver disease is selected from a cholestatic liver disease (chronic cholestatic liver disease), primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma; preferably, the liver disease is primary sclerosing cholangitis (PSC) or primary biliary cirrhosis (PBC); more preferably, the primary sclerosing cholangitis (PSC) or primary biliary cirrhosis (PBC) is accompanied by inflammatory bowel disease as a complication.

49. The combination product, the pharmaceutical composition, the acid-base addition salt, or the compound of formula (I) or the pharmaceutically acceptable salt, the solvate, the hydrate, or the stereoisomer thereof according to claim 47, **characterized in that** the inflammatory bowel disease is a complication arising from primary sclerosing cholangitis (PSC) and/or primary biliary cirrhosis (PBC).

50. A kit, **characterized in that** the kit comprises:
the combination product, the acid-base addition salt, the pharmaceutical composition, or the compound of formula (I) or the pharmaceutically acceptable salt, the solvate, the hydrate, or the stereoisomer thereof according to any one of claims 1-35, and instructions for use, wherein the kit is used for treating and/or preventing a liver disease or inflammatory bowel disease.

51. The kit according to claim 50, **characterized in that** the liver disease is selected from a cholestatic liver disease, primary sclerosing cholangitis (PSC), primary biliary cirrhosis (PBC), progressive familial intrahepatic cholestasis (particularly progressive familial intrahepatic cholestasis types 1, 2, and 3), cystic fibrosis, drug-induced cholestasis or a non-cholestatic liver disease, chronic viral hepatitis (B, C, and D), alcoholic or non-alcoholic steatohepatitis, autoimmune hepatitis, hemochromatosis, Wilson's disease or α-1-antitrypsin deficiency, liver cancer (particularly hepatocellular carcinoma), and cholangiocarcinoma.
